# FASCICULE DE BREVET EUROPEEN

(11) **EP 1 251 125 B1**
(45) Date de publication et mention de la délivrance du brevet: **07.12.2005**
(21) Numéro de dépôt: 02077736.3
(22) Date de dépôt: 21.06.1996
(51) Int. Cl.: C07D 491/22, A61K 31/47, C07D 491/04, C07D 215/18

(54) **Nouveaux analogues de la camptoth-cine, des proc-d-s de pr-paration, leur application comme m-dicaments et les compositions pharmaceutiques les contenant**
Camptothecinanaloge, Verfahren zur ihrer Herstellung, ihre Verwendung als Arzneimittel und diese enthaltende pharmazeutische Zusammensetzungen
New camptothecin analogues, preparation methods thereof, use thereof as drugs, and pharmaceutical compositions containing them

(30) Priorité: 21.06.1995 GB 9512670; 04.03.1996 US 610476
(43) Date de publication de la demande: 23.10.2002
(62) Demande divisionnaire de: 96924010.0
(73) Titulaire: Société de Conseils et de Recherches d'Applications Scientifiques (S.C.R.A.S.), 75016 Paris (FR)
(72) Inventeur: Bigg, Dennis, 91190 Gif-sur-Yvette (FR); Lavergne, Olivier, 91120 Yvette (FR); Pla Rodas, Francesc, Santa Coloma de Farners (ES); Pommier, Jacques, 75015 Paris (FR); Ulibarri, Gérard, Ottawa, ON - Canada K1G 3H7 (CA)
(74) Mandataire: Bourgouin, André

(56) Documents cités:
- WO-A-92/11263
- WO-A-94/11376
- D. L. COMINS ET AL : "Six-step synthesis of (+-)-Camptothecin" JOURNAL OF ORGANIC CHEMISTRY., vol. 59, no. 18, 1994, pages 5120-5121, XP002216662 AMERICAN CHEMICAL SOCIETY. EASTON., US ISSN: 0022-3263
- R. P. HERTZBERG ET AL: "Modification of the hydroxy lactone ring of camptothecin: Inhibition of mammalian topoisomerase I and biological activity" JOURNAL OF MEDICINAL CHEMISTRY, vol. 32, no. 3, pages 715-720, XP002014409 WASHINGTON US

## Description

La camptothécine est un composé naturel qui a été isolé pour la première fois des feuilles et de l'écorce de la plante chinoise appelée *camptotheca acuminata* (voir Wall et ses collaborateurs, J. Amer. Chem. Soc. 88:3888 (1966)). La camptothécine est un composé pentacyclique constitué d' un fragment indolizino[1,2-b] quinoléine fusionné avec une α-hydroxylactone à six chaînons. Le carbone en position 20, qui porte le groupe α-hydroxy, est asymétrique et confère à la molécule un pouvoir rotatoire. La forme naturelle de la camptothécine possède la configuration absolue "S" au carbone 20 et répond à la formule suivante :

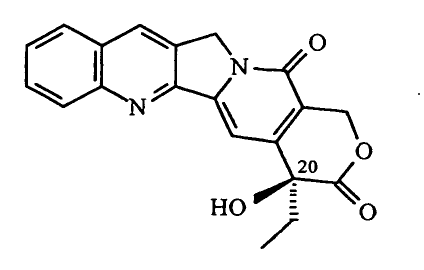

La camptothécine présente une activité anti-proliférative dans plusieurs lignées cellulaires cancéreuses, comprenant les lignées cellulaires de tumeurs humaines du colon, du poumon et du sein (Suffness, M. et collaborateurs : The Alkaloids Chemistry and Pharmacology, Bross, A., ed., Vol. 25, p. 73 (Academic Press, 1985)). On suggère que l'activité anti-proliférative de la camptothécine est en relation avec son activité inhibitrice de la topoisomérase I de l'ADN.

Il a été indiqué que l'α-hydroxylactone était une exigence absolue à la fois pour l'activité *in vivo* et *in vitro* de la camptothécine (Camptothecins : New Anticancer Agents, Putmesil, M., et ses collaborateurs, ed., p. 27 (CRC Press, 1995) ; Wall, M. et ses collaborateurs, Cancer Res. 55:753 (1995) ; Hertzberg et ses collaborateurs, J. Med. Chem. 32:715 (1982) et Crow et ses collaborateurs, J. Med. Chem. 35:4160 (1992)). La présente invention concerne une nouvelle classe de composés de la camptothécine, dans lesquels une β-hydroxylactone remplace l'α-hydroxylactone naturelle de la camptothécine. Les composés selon la présente invention présentent une activité biologique puissante qui est inattendue au regard de l'état de l'art antérieur.

Les nouveaux composés de la camptothécine selon l'invention diffèrent de tout composé de la camptothécine connu, en ce sens qu'ils contiennent une β-hydroxylactone (ou sa forme hydroxycarboxylique ouverte) à la place d'une α-hydroxylactone (ou sa forme hydroxycarboxylique ouverte); ou un sel pharmaceutiquement acceptable de ce dernier. On entend par composé de la camptothécine un composé présentant le même squelette structurel que celui de la camtpothécine (c'est-à-dire un fragment indolizino[1,2-b] quinoléine qui est fusionné à une α-hydroxylactone à six chaînons), avec ou sans autres substitutions chimiques sur la structure du squelette (par exemple les composés de la camptothécine). Différents composés de la camptothécine sont bien connus des spécialistes, comme décrit ci-dessous. On entend par β-hydroxylactone une lactone qui comporte un atome de carbone supplémentaire entre le carbone du carboxyle et le carbone-α portant le groupe hydroxy dans l'α-hydroxylactone. La β-hydroxylactone peut être un cycle à sept chaînons "fermé" ou "ouvert", dans lequel la liaison ester entre le groupe carbonyle et l'atome d'oxygène adjacent est hydrolysée, ce qui se traduit par la formation d'un groupe acide carboxylique et d'un groupe hydroxyle, lesquels groupes peuvent ou non être substitués.

Un composé de la camptothécine selon l'invention peut donc comporter des substitutions sur le fragment indolizino[1,2-5] quinoléine (par exemple pour améliorer la solubilité du composé) ou sur la β-hydroxylactone ouverte ou fermée (par exemple pour améliorer la stabilité du composé). Des exemples de substitutions sur la β-hydroxylactone fermée comprennent une substitution alkyle (par exemple éthyle) sur le carbone-β. Des exemples de substitution sur la β-hydroxylactone ouverte comprennent des substitutions alkyle sur le carbone-β, des substitutions (par exemple une amidation) sur l'acide carboxylique résultant, et des substitutions (par exemple une estérification) ou des suppressions du groupe hydroxyle résultant.

Des synthèses d'analogues de la campthotécine sont proposées dans la littérature : Journal of Organïc Chemistry, vol.59, n°18, 1994 et WO 92/11263 décrivent la synthèse d'analogues de la campthotécine avec une α-hydroxylactone à 6 chaînons par condensation de cycles. Dans WO 94/11376 est décrite la synthèse d'analogues de la campthotécine avec une α-hydroxylactone à 6 ou 7 chaînons. De même des modifications de l'α-hydroxylactone à 6 chaînons sont décrites dans J. Med. Chem., vol.32, 715-720 1989. La présente demande propose la synthèse de nouveaux analogues de la campthotécine comprenant une β-hydroxylactone à 7 chaînons ainsi que des intermédiaires de synthèse.

L'invention a pour objet un procédé de préparation des composés de formule O 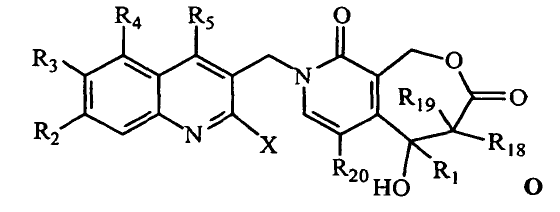 sous forme racémique, d'énantiomère ou toutes combinaisons de ces formes, dans laquelle
- R₁: représente un alkyle inférieur, un alkényle inférieur, un alkynyl inférieur, un haloalkyle inférieur, un alkoxy inférieur alkyle inférieur ou un alkylthio inférieur alkyle inférieur ;
- R₂, R₃ et R₄: représentent, indépendamment, H, halo, halo alkyle inférieur, alkyle inférieur, alkényle inférieur, cyano, cyano alkyle inférieur, nitro, nitro alkyle inférieur, amido, amido alkyle inférieur, hydrazino, hydrazino alkyle inférieur, azido, azido alkyle inférieur, -(CH₂)ₘNR₆R₇, -(CH₂)ₘOR₆, -(CH₂)ₘSR₆, -(CH₂)ₘCO₂R₆, -(CH₂)ₘNR₆C(O)R₈, -(CH₂)ₘC(O)R₈, -(CH₂)ₘOC(O)R₈, -O(CH₂)ₘNR₆R₇, -OC(O)NR₆R₇, -OC(O)(CH₂)ₘCO₂R₆, ou -(CH₂)ₙ[N=X], -OC(O)[N=X], -(CH₂)ₘOC(O)[N=X], aryle ou aryle alkyle inférieur substitué ou non substitué, dans lequel le substituant est un alkyle inférieur, halo, nitro, amino, alkylamino inférieur, haloalkyle inférieur, hydroxy alkyle inférieur, alkoxy inférieur, ou alkoxy inférieur alkyle inférieur, ou R₂ et R₃ forment ensemble une chaîne à 3 ou 4 chaînons, dans laquelle les éléments de la chaîne sont sélectionnés parmi le groupe constitué de CH, CH₂, O, S, N ou NR₉ ;
- R₅: représente H, halo, halo alkyle inférieur, alkyle inférieur, alkoxy inférieur, alkoxy inférieur alkyle inférieur, alkylthio inférieur alkyle inférieur, cycloalkyle, cycloalkyle alkyle inférieur, cyano, cyano alkyle, alkyle inférieur sulphonyl alkyle inférieur, hydroxy alkyle inférieur, nitro, -(CH₂)ₘC(O)R₈, -(CH₂)ₘNR₆C(O)R₈, -(CH₂)ₘNR₆R₇, (CH₂)ₘN(CH₃)(CH₂)ₙNR₆R₇, -(CH₂)ₘOC(O)R₈, -(CH₂)ₘOC(O)NR₆R₇, (CH₂)ₘS(O)_{q}R₁₁, -(CH₂)ₘP(O)R₁₂R₁₃, -(CH₂)₂P(S)R₁₂R₁₃, ou un radical -(CH₂)ₙ[N=X], -OC(O)[N=X], -(CH₂)ₘOC(O)[N=X], aryle ou aryle alkyle inférieur substitué ou non substitué, dans lequel le substituant est un alkyle inférieur, halo, nitro, amino, alkyle inférieur amino, halo alkyle inférieur, hydroxy alkyle inférieur, alkoxy inférieur ou alkoxy inférieur alkyle inférieur ;
- R₆ et R₇: représentent, indépendamment, H, un alkyle inférieur, hydroxy alkyle inférieur, alkyle inférieur amino alkyle inférieur, amino alkyle inférieur, cycloalkyle, cycloalkyle alkyle inférieur, alkényle inférieur, alkoxy inférieur alkyle inférieur, halo alkyle inférieur, le radical substitué ou non substitué aryle ou aryle alkyle inférieur, dans lequel le substituant est un alkyle inférieur, halo, nitro, amino, alkyle inférieur amino, halo alkyle inférieur, hydroxy alkyle inférieur, alkoxy inférieur, ou alkoxy inférieur alkyle inférieur ;
- R₈: représente H, un alkyle inférieur, hydroxy alkyle inférieur, amino, alkyle inférieur amino, alkyle inférieur amino alkyle inférieur, amino alkyle inférieur, cycloalkyle, cycloalkyle alkyle inférieur, alkényle inférieur, alkoxy inférieur, alkoxy inférieur alkyle inférieur, halo alkyle inférieur, ou le radical substitué ou non substitué aryle ou aryle alkyle inférieur, dans lequel le substituant est un alkyle inférieur, halo, nitro, amino, alkyle inférieur amino, halo alkyle inférieur, hydroxy alkyle inférieur, alkoxy inférieur, ou alkoxy inférieur alkyle inférieur ;
- R₉: représente H, un alkyle inférieur, halo alkyle inférieur, aryle, ou aryle substitué par un ou plusieurs groupes choisis parmi le radical alkyle inférieur, halo, nitro, amino, alkyle inférieur amino, halo alkyle inférieur, hydroxy alkyle inférieur, alkoxy inférieur, ou alkoxy inférieur alkyle inférieur ;
- R₁₀: représente H, un alkyle inférieur, halo alkyle inférieur, alkoxy inférieur, aryle, ou aryle substitué par un ou plusieurs groupes choisis parmi le radical alkyle inférieur, halo alkyle inférieur, hydroxy alkyle inférieur, ou alkoxy inférieur alkyle inférieur ;
- R₁₁: représente un alkyle inférieur, aryle, -(CH₂)ₘOR₁₄, -(CH₂)ₘSR₁₄, -(CH₂)₂NR₁₄R₁₅ ou -(CH₂)ₘ[N=X] ;
- R₁₂ et R₁₃: représentent, indépendamment, un alkyle inférieur, aryle, alkoxy inférieur, aryloxy ou amino ;
- R₁₄ et R₁₅: représentent, indépendamment, H, un alkyle inférieur ou aryle ;
- R₁₈ et R₁₉: représentent, indépendamment, H, halo, alkyl inférieur, alkoxy inférieur ou hydroxy ;
- R₂₀: représente H ou halo ;
- m: est un nombre entier compris entre 0 et 6 ;
- n: est 1 ou 2 ; et
- q: représente un nombre entier de 0 à 2 ;
- [N=X]: représente un groupe hétérocyclique de 4 à 7 chaînons, X représentant la chaîne nécessaire pour compléter ledit groupe hétérocyclique et sélectionnée dans le groupe constitué de O, S, CH₂, CH, N, NR₉ et COR₁₀ ; et

X représente un atome d'halogène, ou un sel pharmaceutiquement acceptable de ce dernier, étant entendu que le terme inférieur en référence aux groupes alkyle, alkylthio et alkoxy signifie un groupe comportant de 1 à 6 atomes de carbone et en référence aux groupes alkényle ou alkynyle, un groupe comportant de 2 à 6 atomes de carbone,
caractérisé en ce que l'on couple un composé de formule générale M 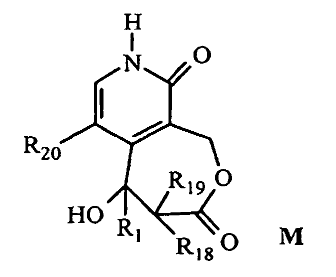 dans laquelle R₁, R₁₈, R₁₉ et R₂₀ ont la signification indiquée ci-dessus, avec un 2-halo-3-quinoléine-méthanol de formule générale N 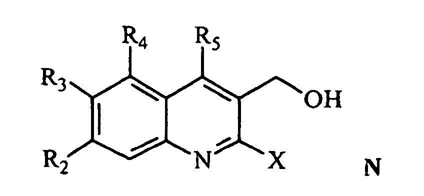 dans laquelle R₂, R₃, R₄, R₅ et X ont la signification indiquée ci-dessus, pour donner le composé de formule O.

De préférence, l'invention a pour objet un procédé de préparation tel défini ci-dessus, caractérisé en ce que
- R₁: représente un alkyle inférieur, alkényle inférieur, halo alkyle inférieur ou alkoxy inférieur alkyle inférieur ;
- R₂, R₃ et R₄: représentent, indépendamment, H, un halo, halo alkyle inférieur, alkyle inférieur, nitro, amido, amido alkyle inférieur, hydrazino, hydrazino alkyle inférieur, azido, azido alkyle inférieur, -(CH₂)ₘNR₆R₇, -(CH₂)ₘOR₆, -(CH₂)ₘSR₆, -(CH₂)ₘC(O)R₈, -OC(O)NR₆R₇, -(CH₂)ₙ[N=X] ou -(CH₂)ₘOC(O)[N=X] substitué ou non substitué, ou -OC(O)[N=X], ou R₂ et R₃, ensemble, forment une chaîne à 3 ou 4 chaînons, dans laquelle lesdits éléments de la chaîne sont choisis dans le groupe constitué de CH, CH₂, O, S, N ou NR₉ ;
- R₅: représente H, halo, halo alkyle inférieur, alkyle inférieur, alkoxy inférieur, alkoxy inférieur alkyle inférieur, alkylthio inférieur alkyle inférieur, hydroxy alkyle inférieur, nitro, -(CH₂)ₘC(O)R₈, -(CH₂)ₘNR₆C(O)R₈, -(CH₂)ₘNR₆R₇, -(CH₂)ₘN(CH₃)(CH₂)ₙNR₆R₇, -(CH₂)ₘOC(O)R₈, -(CH₂)ₘOC(O)NR₆R₇, -(CH₂)ₙ[N=X] ou -OC(O)[N=X] substitué ou non substitué, ou -(CH₂)ₘOC(O)[N=X] ;
- R₆ et R₇: représentent, indépendamment, H, un alkyle inférieur, hydroxy alkyle inférieur, alkyle inférieur amino alkyle inférieur, amino alkyle inférieur, cycloalkyle, cycloalkyle alkyle inférieur, alkoxy inférieur alkyle inférieur, aryle, aryle alkyle inférieur ou halo alkyle inférieur ;
- R₈: représente H, un alkyle inférieur, hydroxy alkyle inférieur, alkyle inférieur amino, alkyle inférieur amino alkyle inférieur, amino alkyle inférieur, cycloalkyle, cycloalkyle alkyle inférieur, alkényle inférieur, alkoxy inférieur, alkoxy inférieur alkyle inférieur, halo alkyle inférieur, aryle ou aryle alkyle inférieur ;
- R₉: représente H, un alkyle inférieur, ou un halo alkyle inférieur ;
- R₁₀: représente H, un alkyle inférieur, halo alkyle inférieur, ou alkoxy inférieur ;
- R₁₈, R₁₉ et R₂₀: représentent H.

De préférence également, l'invention a pour objet un procédé de préparation tel que défini ci-dessus, caractérisé en ce que R₁ représente le groupe éthyle.

De préférence également, l'invention a pour objet un procédé de préparation tel que défini ci-dessus, caractérisé en ce que R₂ et R₃ représentent, indépendamment, H, un alkyle inférieur, halo, halo alkyle inférieur ou -(CH₂)ₘOR₆, ou R₂ et R₃, ensemble, forment un méthylènedioxy ou un éthylènedioxy; et R₄ et R₅ représentent, indépendamment, H, un alkyle inférieur, -(CH₂)ₘNR₆R₇ ou -(CH₂)ₙ[N=X] non substitué ou substitué par un alkyle inférieur.

De préférence également, l'invention a pour objet un procédé de préparation tel que défini ci-dessus, caractérisé en ce que R₄ représente H ou -(CH₂)ₘNR₆R₇ dans lequel R₆ et R₇ représentent, indépendamment, H ou un alkyle inférieur, et R₅ représente H, un alkyle inférieur ou -(CH₂)ₙ[N=X] substitué ou non substitué et [N=X] représente le groupe pipérazinyle ou morpholinyle et ledit substituant est un alkyle inférieur.

De manière très préférentielle, l'invention a pour objet un procédé de préparation tel que défini ci-dessus, caractérisé en ce que R₂ représente H ou un halo ; et R₃ représente H, un alkyle inférieur, halo, ou OR₆ dans lequel R₆ représente H, un alkyle inférieur ou un aryle alkyle inférieur.

De manière très préférentielle également, l'invention a pour objet un procédé de préparation tel que défini ci-dessus, caractérisé en ce que R₂ représente H, chloro ou fluoro ; et R₃ représente H, fluoro, chloro, méthyle ou méthoxy.

Tel qu'il est utilisé ici, le terme inférieur en référence aux groupes alkyl, alkylthio et alkoxy désigne des groupes hydrocarbonés aliphatiques saturés, linéaires, ou ramifiés, comportant de 1 à 6 carbones, comme par exemple méthyle, éthyle, propyle, isopropyle, butyle, t-butyle, méthylthio, éthylthio, méthoxy et éthoxy. En référence aux groupes alkényle ou alkynyle, le terme inférieur désigne des groupes comportant 2 à 6 atomes de carbone et une ou plusieurs doubles ou triples liaisons, comme par exemple les groupes vinyle, allyl, isopropényle, pentènyle, hexanyle, propènyle éthynyle, propynyle et butynyle. Le terme cycloalkyle désigne un cycle de 3 à 7 carbones, comme par exemple les groupes cyclopropyle, cyclobutyle, cyclopentyle ou cyclohexyle. Le terme aryl désigne un composé hydrocarboné mono-, di ou tricyclique avec au moins un cycle aromatique, chaque cycle contenant au maximum 7 chaînons, comme par exemple phényle, naphtyle, anthracyle, biphényle ou indényle . Le terme halo signifie chloro, bromo, iodo ou fluoro. Les radicaux correspondant aux expressions halo alkyle inférieur, cyano alkyle inférieur, nitro alkyle inférieur, amido alkyle inférieur, hydrazino alkyle inférieur, azido alkyle inférieur, aryle alkyle inférieur, hydroxy alkyle inférieur, alkoxy inférieur alkyle inférieur, alkylthio inférieur alkyle inférieur, et alkyle inférieur sulphonyle alkyle inférieur sont substitués, respectivement, par un à trois groupes halo, cyano, nitro, amido, hydrazino, azido, aryle, hydroxy, alkoxy inférieur, alkylthio inférieur ou sulphonyle inférieur. Le radical alkyle inférieur amino peut contenir un ou deux groupes alkyle inférieur, et représenter par exemple NHCH₃, NHCH₂CH₃, N(CH₃)₂, ou N(CH₃)(CH₂CH₃). Des exemples de [N=X] comprennent les groupes pipéridinyle, morpholinyle, piperizinyle et imidazolyle.

Comme cela est observé pour la camptothécine, l'atome de carbone portant la fonction hydroxy dans la β-hydroxylactone ou le groupe β-hydroxycarboxylate des composés, selon la présente invention, est asymétrique. Par conséquent, les composés selon la présente invention ont deux formes énantiomères possibles, c'est-à-dire sous les configurations "R" et "S". La présente invention inclut les deux formes éniantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

L'invention a également pour objet des composés de formule O 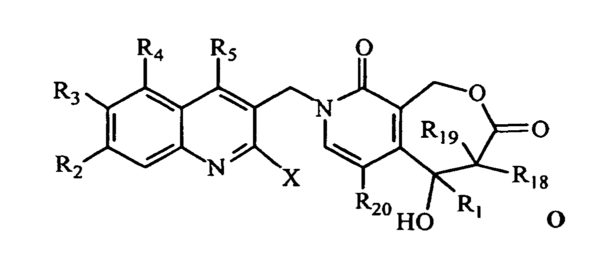 dans laquelle R₁, R₂, R₃, R₄, R₅, R₁₈, R₁₉, R₂₀ et X ont la signification indiquée ci-dessus.

L'invention a également pour objet l'utilisation des composés de formule O tels que définis ci-dessus, pour la préparation des composés de formule I 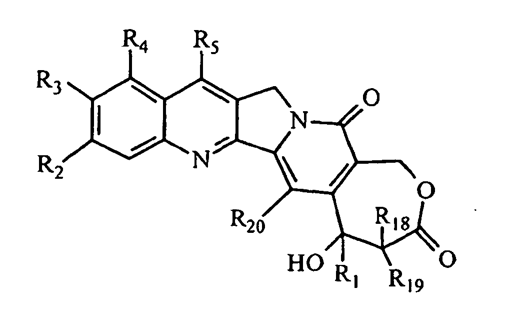 dans laquelle R₁, R₂, R₃, R₄, R₅, R₁₈, R₁₉ et R₂₀ ont la signification indiquée ci-dessus, ou un sel thérapeutiquement acceptable de ces derniers,
caractérisé en ce que l'on cyclise le composé de formule générale O tel que défini ci-dessus, pour obtenir le composé de formule générale 1telle que définie ci-dessus.

De préférence, l'invention a pour objet l'utilisation des composés de formule O tels que définis ci-dessus pour la préparation d'un composé d'un composé de formule (I), caractérisé en ce que ledit composé répond à la formule suivante : 5-éthyl-9,10-difluoro-4,5-dihydro-5-hydroxy-1*H-*oxepino[3',4':6,7]indolizino[1,2-*b*]quinoléine-3,15(4*H*,13*H*)-dione.

Dans le procédé ci-dessus, les groupes R₁, R₂, R₃ et R₄ peuvent être protégés si nécessaire selon les méthodes classiques de protection (Greene, T., Protective Groups in Organic Synthesis 10-86 (John Wiley & Sons 1981)). La formation du composé O à partir des composés de formule générale M et N s'effectue par un traitement connu de l'homme de l'art sous le nom de réaction de Mitsunobu (se référer à Mitsunobu, O. et coll. *Synthesis,* p.1 (1981)). Il s'agit de déplacer la fonction hydroxyle du composé N par un nucléophile tel que le composé M, ou un dérivé déprotoné de ce dernier, par un traitement avec une phosphine, par exemple la triphénylphosphine, et un dérivé azodicarboxylate, par exemple l'azodicarboxylate de diéthyle, dans un solvant aprotique tel que, par exemple, le tétrahydrofurane ou le *N,N*-diméthylformamide. La cyclisation du composé O s'effectue de préférence en présence d'un catalyseur palladié (par exemple le diacétate de palladium) dans des conditions basiques (fournies par exemple par un acétate alcalin éventuellement combiné à un agent de transfert de phase tel que par exemple le bromure de tétrabutylammonium), dans un solvant aprotique tel que l'acétonitrile ou le *N*,*N*-diméthylformamide, à une température comprise entre 50° C et 120° C (R. Grigg et coll., *Tetrahedron* 46, page 4003 (1990)).

Les composés de formule générale M sont nouveaux. Ils peuvent être préparés selon un procédé caractérisé en ce que
- l'on protège le carbonyle d'une pyridine de formule générale 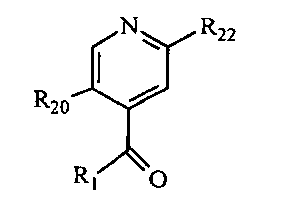 dans laquelle R₁ et R₂₀ ont la signification indiquée ci-dessus et R₂₂ représente un atome d'halogène ou un alkoxy inférieur, par une fonction acétal, pour donner le composé de formule générale F 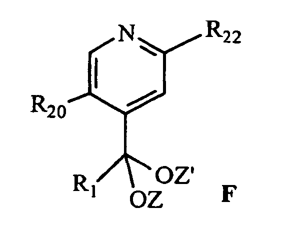 dans laquelle R₁, R₂₀ et R₂₂ ont la signification indiquée ci-dessus et les groupes Z et Z' représentent, indépendamment, un alkyl inférieur ou forment ensemble une chaîne hydrocarbonée saturée de 2 à 4 carbones ;
- l'on introduit dans le composé de formule générale F une fonction hydroxyméthyle pour obtenir un composé de formule générale G 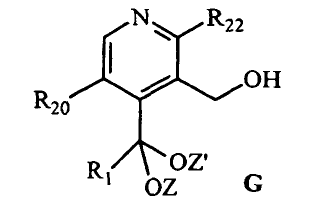 dans laquelle R₁, R₂₀, R₂₂, Z et Z' ont la signification indiquée ci-dessus,
- puis l'on protège la fonction alcoolique du composé de formule générale G pour donner un composé de formule générale H 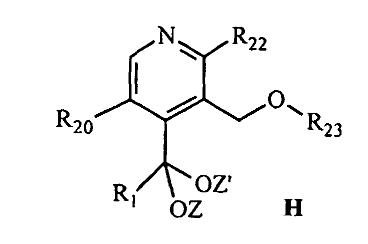 dans laquelle R₁, R₂₀, R₂₂, Z et Z' ont la signification indiquée ci-dessus et R₂₃ représente un groupe protecteur de la fonction alcool,
- l'on déprotège l'acétal du composé de formule générale H pour donner le composé de formule générale I' 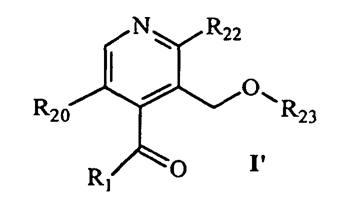 dans laquelle R₁, R₂₀, R₂₂ et R₂₃ ont la signification indiquée ci-dessus,
- l'on traite par un agent alkylant fonctionnalisé le composé de formule I' pour donner un β-hydroxyester de formule générale J 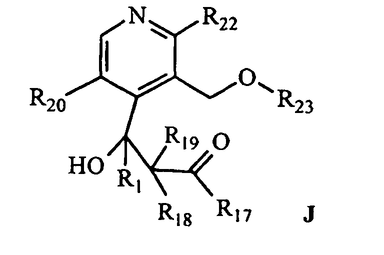 dans laquelle R₁, R₁₈, R₁₉, R₂₀, R₂₂ et R₂₃ ont la signification indiquée ci-dessus et R₁₇ représente -OR₆ ou -NR₆R₇ et R₆ et R₇ sont tels que définis ci-dessus,
- l'on clive le groupe protecteur R₂₃ du composé de formule générale J, pour donner un composé de formule générale K, 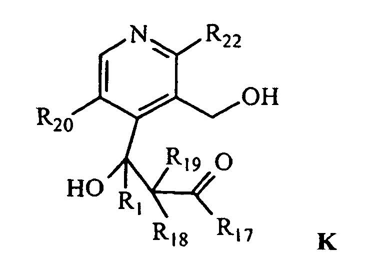 dans laquelle R₁, R₁₈, R₁₉, R₂₀ et R₂₂ ont la signification indiquée ci-dessus, et R₁₇ représente OR₆ ou NHR₆ et R₆ représente H, un alkyle inférieur, cycloalkyle, cycloalkyle alkyle inférieur, alkényle inférieur, alkoxy inférieur alkyle inférieur, ou aryl ou aryle alkyle inférieur,
- l'on cyclise le composé de formule générale K en composé de formule générale L 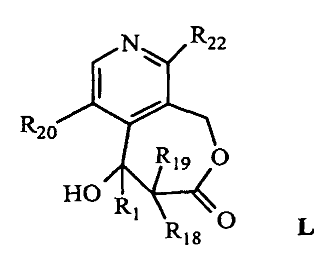 dans laquelle R₁, R₁₈, R₁₉, R₂₀ et R₂₂ ont la signification indiquée ci-dessus, et enfin
- l'on transforme le radical R₂₂ du composé L en carbonyle, pour obtenir le composé de formule générale M 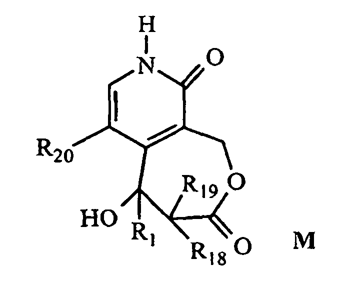 dans laquelle R₁, R₁₈, R₁₉ et R₂₀ ont la signification indiquée ci-dessus.

La fonction carbonyle d'une 4-acyl-2-halopyridine (obtenue par exemple selon Lamattina, J.L. *J. Hétérocyclic Chem. 20,* p. 553 (1983)) est protégée de préférence par une fonction acétal, de préférence acétal cyclique, selon des condition classiques connues de l'homme de l'art (Greene, T., Protective Groups in Organic Synthesis 10-86 (John Wiley & Sons 1981)).L'intermédiaire ainsi obtenu est traité par un alcoolate de sodium ou de potassium dans un solvant aprotique (par exemple l'acétonitrile), ou dans l'alcool dont est dérivé l'alcoolate, à une température comprise entre 0° C et 100° C pour donner le composé de formule générale F. Ce dernier peut être lithié en position 3 par traitement par un aryl- ou alkyl-lithium (par exemple mésityl-lithium) dans un solvant éthéré tel que le tétrahydrofurane à une température comprise entre -100° C et 0° C. A l'intermédiaire lithié ainsi obtenu, on ajoute un électrophile formylant tel que le *N*,*N*-diméthylformamide, et l'aldéhyde ainsi obtenu, après hydrolyse, est traité par un agent réducteur tel que le borohydrure de sodium pour donner le composé de formule générale G. La protection de la fonction alcool du composé G s'effectue selon des conditions classiques connues de l'homme de l'art, pour obtenir un composé de formule générale H. Des exemples de groupes protecteurs de la fonction alcool comprennent ceux qui forment les éthers [c'est-à-dire, méthyle, méthoxyméthyle, tétrahydropyranyl, 2-méthoxyéthoxy méthyle, benzyloxyméthyle, t-butyl, et benzyl (substitué ou non)], et les esters (c'est-à-dire formate, acétate et isobutyrate). Pour d'autres exemples de groupes protecteurs d'hydroxyl primaires, on peut se référer à Greene, T., Protectives Groups in Organic Synthesis, 10-86 (John Wiley & Sons, 1981). La déprotection du composé de formule générale H pour donner le composé de formule générale I' s'effectue dans des conditions sélectives maintenant l'intégrité du radical R₂₃, par exemple, par traitement dans des conditions acides (par exemple par l'acide trifluoroacétique). Les conditions sélectives de protection et de déprotection des groupes fonctionnels sont connues de l'homme de l'art (Greene, T., Protective Groups in Organic Synthesis 10-86 (John Wiley & Sons 1981)). Le traitement du composé I' par un agent alkylant fonctionnalisé pour donner un β-hydroxyester de formule générale J, peut être réalisée à l'aide d'un énolate de lithium ou d'un dérivé zincique d'un ester carboxylique dans un solvant aprotique anhydre, par exemple le tétrahydrofurane.Le groupe protecteur R₂₃ du composé de formule générale J, est clivé pour donner un composé de formule générale K, dans des conditions de déprotection connues de l'homme de l'art. Par exemple, lorsque R₂₃ est un groupe benzyle, on peut soumettre une solution alcoolique du composé de formule générale J additionnée d'un catalyseur palladié à une atmosphère d'hydrogène sous une pression de 0,5 à 10 Bar. La cyclisation du composé de formule générale K ainsi obtenu peut être effectuée dans des conditions acides (par exemple par traitement par l'acide trifluoroacétique, ou le gaz chlorhydrique dissout dans un solvant anhydre tel que le dichlorométhane ou le dioxane) pour donner un cycle β-hydoxylactonique à sept chaînons tel que dans le composé de formule générale L. Les composés de formule générale L peuvent être transformés en pyridones de formule générale M, par exemple, par un traitement à l'acide chlorhydrique à chaud, ou par un traitement à l'iodure de triméthylsilyle.

Les 2-halo-3-quinoléine-méthanols de formule générale N peuvent être obtenus à partir d'acétanilides de formule générale P 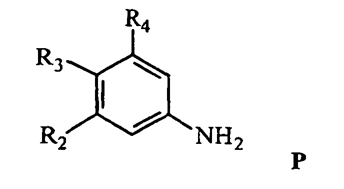 dans laquelle R₂, R₃ et R₄ ont la signification indiquée dans la formule générale des composés (I). Dans les procédés ci-dessous, les groupes R₂, R₃ et R₄ peuvent être protégés si nécessaire selon les méthodes classiques de protection (Greene, T., Protective Groups in Organic Synthesis 10-86 (John Wiley & Sons 1981)).

Les composés de formule N peuvent donc être obtenus selon le procédé suivant : les dites anilines de formule P sont *N*-acétylées par traitement avec un agent acétylant tel que par exemple l'anhydride acétique. Les acétanilides ainsi obtenus sont traités à une température comprise entre 50° C et 100° C, de préférence 75° C, par un réactif connu de l'homme de l'art sous de nom de réactif de Vilsmeyer (obtenu par l'action de l'oxychlorure de phosphoryle sur la *N,N*-diméthylformamide à une température comprise entre 0°C et 10°C) pour donner le 2-chloro-3-quinoléinecarbaldéhyde correspondant (se référer, par exemple à Meth-Cohn, et coll. *J. Chem. Soc*., *Perkin Trans. I* p.1520 (1981) ; Meth-Cohn, et coll. *J*. *Chem. Soc*., *Perkin Trans. I* p.2509 (1981) ; et Nakasimhan et coll. *J*. *Am. Chem. Soc*., *112*, p.4431 (1990)). Le chlore en position 2 des 2-chloro-3-quinoléinecarbaldéhydes peut être substitué par de l'iode ou du brome en chauffant le produit dans un solvant inerte tel que l'acétonitrile en présence d'un sel d'iode ou de brome (par exemple l'iodure de sodium ou le tétrabutylammonium bromure). Une trace d'acide tel que l'acide chlorhydrique concentré peut être nécessaire pour catalyser cette transformation. Les 2-halo-3-quinoléinecarbaldéhydes sont facilement réduits en 2-halo-3-quinoléineméthanols correspondants de formule générale N, dans des conditions classiques connues de l'homme de l'art telles que le traitement dans un solvant alcoolique (par exemple le méthanol) par du borohydrure de sodium à une température comprise entre 0° C et 40° C.

Les composés de formule N peuvent également être obtenus selon le procédé suivant : les anilines de formule générale P telles que définies ci-dessus sont acylées par réaction avec un nitrile (tel que le chloroacétonitrile ou le propionitrile) en présence de trichlorure de bore et d'un autre acide de Lewis tel que le trichlorure d'aluminium, le tétrachlorure de titane ou le chlorure de diéthylaluminium dans un solvant aprotique ou un mélange de solvant aprotique, suivie d'une hydrolyse (*cf*. Sugasawa, T, et coll*. J. Am. Chem. Soc. 100,* p. 4842 (1978)). L'intermédiaire ainsi obtenu est ensuite traité par le chlorure d'éthylmalonyle dans un solvant aprotique tel que l'acétonitrile en présence d'une base telle que la triéthylamine, puis traité par un alcoolate alcalin, par exemple le méthylate de sodium dans le méthanol, pour donner un 2-hydroxy-3-quinoléinecarboxylate d'éthyle substitué en position 4. Ce dernier est transformé en 2-chloro-3-quinoléinecarboxylate d'éthyle par un traitement avec de l'oxychlorure de phosphoryle. Lorsque la position 4 de la quinoléine porte un groupe chlorométhyle, on peut effectuer une substitution nucléophile par traitement avec une amine secondaire telle que par exemple la diméthylamine, la N-méthylpipérazine, la morpholine ou la pipéridine. Le 2-chloro-3-quinoléinecarboxylate d'éthyle est ensuite réduit par l'hydrure de diisobutylaluminium dans un solvant aprotique que le dichlorométhane pour donner le 2-chloro-3-quinoléineméthanol de formule générale N. Des analogues des composés intermédiaires (N) ont été décrits dans la littérature et en particulier dans la demande PCT 95/05427.

Certains composés (I) de l'invention peuvent être préparés sous la forme de sels pharmaceutiquement acceptables selon les méthodes usuelles. Des sels acceptables comprennent, à titre d'exemple et de façon non limitative, des sels d'addition d'acides inorganiques tels que chlorhydrate, sulfate, phosphate, diphosphate, bromhydrate et nitrate ou d'acides organiques tels que acétate, maléate, fumarate, tartrate, succinate, citrate, lactate, méthane sulfonate, p-toluènesulfonate, pamoate, salicylate, oxalate et stéarate. Entrent également dans le champ d'application de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Pharmaceutical Salts", J. Pharm. Sci. 66:1 (1977).

Les composés (I) de la présente invention possèdent d'intéressantes propriétés pharmacologiques. C'est ainsi que les composés de la présente invention ont une activité inhibitrice de la topoisomérase I et/ou II et une activité anti-tumorale. L'état de la technique suggère que les composés de l'invention présentent une activité anti-parasitique et/ou anti-virale. Les composés de la présente invention peuvent ainsi être utilisés dans différentes applications thérapeutiques. Les composés (I) peuvent inhiber la topoisomérase, par exemple de type I et/ou II, chez un patient, par exemple un mammifère tel que l'homme, par administration à ce patient d'une quantité thérapeutiquement efficace d'un composé de formule (I).

Les composés (I) de l'invention possèdent également une activité anti-tumorale. Ils peuvent être utilisés pour le traitement des tumeurs, par exemple des tumeurs exprimant une topoisomérase, chez un patient par administration à ce dernier d'une quantité thérapeutiquement efficace d'un composé de formule (I). Des exemples de tumeurs ou de cancers comprennent les cancers de l'oesophage, de l'estomac, des intestins, du rectum, de la cavité orale, du pharynx, du larynx, du poumon, du colon, du sein, du cervix uteri, du corpus endométrium, des ovaires, de la prostate, des testicules, de la vessie, des reins, du foie, du pancréas, des os, des tissus conjonctifs, de la peau, des yeux, du cerveau et du système nerveux central, ainsi que le cancer de la thyroïde, la leucémie, la maladie de Hodgkin, les lymphomes autres que ceux de Hodgkin, les myélomes multiples et autres.

Ils peuvent également être utilisés pour le traitement des infections parasitiques par l'inhibition des hémoflagellates (par exemple dans la trypanosomie ou les infections leiskmania) ou par inhibition de la plasmodie (comme par exemple dans la malaria), mais aussi le traitement des infections ou maladies virales.

Ces propriétés rendent les produits de formule I aptes à une utilisation pharmaceutique.

Les compositions pharmaceutiques contenant un composé (I) ou un sel additif d'acide pharmaceutiquement acceptable de celui-ci, en association avec un support pharmaceutiquement acceptable suivant le mode d'administration choisie (par exemple orale, intraveineuse, intrapéritonéales, intramusculaires, trans-dermique ou sous-cutanée), peut être sous forme solide, liquide, de liposome ou de micelles lipidiques.

La composition pharmaceutique peut être sous forme solide comme, par exemple, les poudres, pilules, granules, comprimés, liposomes, gélules ou suppositoires. La pilule, le comprimé ou la gélule peuvent être revêtus d'une substance capable de protéger la composition de l'action de l'acide gastrique ou des enzymes dans l'estomac du sujet pendant une période de temps suffisante pour permettre à cette composition de passer non digérée dans l'intestin grêle de ce dernier. Le composé peut aussi être administré localement, par exemple à l'emplacement même de la tumeur. Le composé peut aussi être administré selon le processus de la libération prolongée (par exemple une composition à libération prolongée ou une pompe d'infusion). Les supports solides appropriés peuvent être, par exemple, le phosphate de calcium, le stéarate de magnésium, le carbonate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire. Les compositions pharmaceutiques contenant un composé de l'invention peuvent donc également se présenter sous forme liquide comme, par exemple, des solutions, des émulsions, des suspensions ou une formulation à libération prolongée. Les supports liquides appropriés peuvent être, par exemple, l'eau, les solvants organiques tels que le glycérol ou les glycols tel que le polyéthylène glycol, de même que leurs mélanges, dans des proportions variées, dans l'eau.

La dose d'un composé (I) selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnés ici sont incorporés par référence.

Les exemples suivants sont présentés pour illustrer les procédures ci-dessus et ne doivent en aucun cas être considérés comme une limite à la portée de l'invention.

### PARTIE EXPÉRIMENTALE :

### Exemple 1 : β-éthyl-β-hydroxy-γ-(8-hydroxyméthyl-9-oxo (11H)-indolizino-[1,2-b] quinoléine-7-yl)-propionate de tert-butyle

### 1.a. 4-éthyl-3,4-dihydroxy-1H-pyrano [3',4':6,7] indolizino [1,2-b] quinoléine-14 (4H,12H)-one

On ajoute par portions du borohydrure de sodium (14 g, 370 mmol) à une suspension de (*S*)-(+)-camptothécine (14 g, 40 mmol, que l'on peut obtenir de différentes sources commerciales telles que Aldrich Chemical Co. (Milwaukee, WI)), dans le méthanol (750 ml) et on chauffe doucement le mélange résultant jusqu'à 55° C afin d'obtenir une solution limpide que l'on agite ensuite pendant 16 heures à la température ambiante. Le solvant est ensuite évaporé sous pression réduite, le résidu est repris dans l'eau (250 ml), neutralisé par addition d'acide acétique (21 ml) et laissé au repos 2 heures à 4° C. La suspension résultante est filtrée et lavée successivement avec de l'eau froide, de l'acétone et du diéthyl éther, ce qui permet d'obtenir après séchage sous pression réduite le composé recherché sous forme d'un solide blanc, p.f. 280° C.

### 1.b. 8-formyloxyméthyl-7-propionylindolizino [1,2-b] quinoléine-9 (11H)-one

On ajoute goutte à goutte une solution de métapériodate de sodium (14 g, 65 mmol) dans l'eau (140 ml) à une suspension de 4-éthyl-3,4-dihydroxy-1H-pyrano [3', 4':6,7] indolizino [1,2-*b*] quinoléine-14 (4*H*,12*H*)-one (13,4 g, 38 mmol) dans de l'acide acétique glacial (720 ml) et on agite la solution résultante pendant une heure à température ambiante. Le mélange réactionnel est ensuite versé dans un mélange glace/eau (650 ml) et la suspension résultante est agitée pendant une demi-heure puis filtrée et lavée successivement avec de l'eau, de l'alcool isopropylique et du étherdiéthylique, ce qui permet d'obtenir, après séchage sous pression réduite, le composé recherché (11,5 g) sous forme d'un solide jaune pâle, p.f. > 200° C (d).

### 1.c. β-éthyl-β-hydroxy-γ-(8-hydroxyméthyl-9-oxo-(11H)-indolizino-[1,2-b] quinoléine-7-yl)-propionate de tert-butyle

Une suspension de zinc (6,5 g, 100 mmol) agitée avec un agitateur magnétique dans du diéthyléther anhydre (50 ml) sous argon, est activée, par addition goutte à goutte de chlorotriméthylsilane (0,75 ml, 5,7 mmol). On agite encore 15 minutes à température ambiante puis on chauffe à reflux. Le bain de chauffage est ensuite enlevé et du bromoacétate de tert-butyle (15 ml, 100 mmol) est ajouté goutte à goutte à une vitesse assurant le maintien au reflux. Le chauffage externe est remis et poursuivi pendant encore une heure. La solution éthérée résultante du réactif de Reformatsky est laissée à refroidir jusqu'à la température ambiante puis transférée au moyen d'une canule dans une suspension de 8-formyloxyméthyl-7-propionylindolizino [1,2-*b*] quinoléine-9 (11*H*)-one (1,6 g, 4,7 mmol) dans du tétrahydrofuranne anhydre (40 ml) sous argon. Le mélange réactionnel est agité au reflux pendant une heure, puis laissé à refroidir jusqu'à la température ambiante, et la réaction est arrêtée par ajout de chlorure d'ammonium saturé (100 ml) et on extrait avec du chloroforme (3 x 100 ml). Les extraits chloroformiques combinés sont séchés sur sulfate de sodium, évaporés, et le résidu est purifié par chromatographie sur colonne de gel de silice (1-2 % MeOH/CH₂Cl₂), ce qui permet d'obtenir 0,64 g du composé recherché (31 %) sous forme d'un solide jaune pâle, p.f. 146-149° C.
RMN-¹H (CDCl₃) : 0,93 (t, 3H) ; 1,37 (s, 9H) ; 1,99 (m, 2H) ; 2,97 (dd, 2H) ; 3,5 (se, 1H) ; 5,10 (s, 2H) ; 5,24 (s, 2H) ; 7,40 (s,1H) ; 7,59 (t, 1H) ; 7,83 (t, 1H) ; 7,90 (d, 1H) ; 8,20 (d, 1H); 8,34 (s, 1H).
RMN-C¹³ (CDCl₃) : 8,18 ; 27,90; 34,59; 45,34; 49,91 ; 58,55 ; 77,39 ; 82,42; 100,52 ; 127,67 ; 127,97 ; 128,10 ; 128,64 ; 129,44 ; 129,79 ; 130,42 ; 130,99 ; 142,86 ; 148,69 ; 152,75 ; 155,16 ; 162,38 ; 172,24.
IR (KBr) : 764 ; 1016 ; 1157 ; 1580 ; 1651 ; 1726.

### Exemple 2 : β-éthyl-β-hydroxy-γ-(8-hydroxyrnéthyl-9-oxo (11H)-indolizino-[1,2-b] quinoléine-7-yl)-propionate d'éthyle

On chauffe à reflux sous argon une suspension de zinc (500 mg, 7,64 mmol) et de la 8-formyloxyméthyl-7-propionylindolizino [1,2-*b*] quinoléine-9 (11*H*)-one (400 mg, 1,15 mmol) dans du tétrahydrofuranne anhydre (20 ml) contenant 10 mg d'hydroquinone. Le bain de chauffage est enlevé et la réaction exothermique est initiée par l'addition d'une goutte de bromoacétate d'éthyle et d'un petit cristal d'iode. Le reflux est maintenu par addition goutte à goutte de bromoacétate d'éthyle (500 µl, 4,48 mmol) puis le mélange réactionnel est encore chauffé à reflux pendant une heure. Après refroidissement à la température ambiante, la réaction est arrêtée par ajout de chlorure d'ammonium saturé (10 ml) et du méthanol (30 ml). Le mélange résultant est agité 5 minutes puis filtré et évaporé. Le résidu est dissous dans du dichlorométhane (30 ml), lavé avec de l'eau et séché sur sulfate de sodium. On procède ensuite à l'élimination du solvant et à une purification par chromatographie sur colonne (SiO₂, CH₂Cl₂/MeOH 98/2), ce qui donne 230 mg (49 %) du composé recherché sous forme d'un solide jaune, p.f. 157-161° C.
RMN-¹H (CDCl₃) : 0,93 (t, 3H) ; 1,20 (t, 3H) ; 2,02 (m, 2H) ; 3,07 (dd, 2H) ; 4,11 (q, 2H) ; 4,9 (se, 1H) ; 5,08 (s, 2H) ; 5,23 (s, 2H) ; 7,45 (s,1H) ; 7,62 (t, 1H) ; 7,80 (t,1H) ; 7,90 (d, 1H) ; 8,22 (d, 1H) ; 8,36 (s,1H).
RMN-C¹³ (CDCl₃) : 8,09 ; 14,01 ; 34,67 ; 44,85 ; 49,94 ; 58,31 ; 61,09 ; 77,21 ; 100,78 ; 127,78 ; 127,96 ; 128,11 ; 128,72 ; 129,16 ; 129,65 ; 130,60 ; 131,32 ; 142,76 ; 148,28 ; 152,55 ; 155,09 ; 162,22 ; 172,59.
IR (KBr) : 766 ; 1009 ; 1184 ; 1582 ; 1647 ; 1750.

### Exemple 3 : 5-éthyl-4,5-dihydro-5-hydroxy-1H-oxepino [3',4':6,7]-indolizine [1,2-b] quinoléine-3,15 (4H,13H)-dione

Du β-éthyl-β-hydroxy-γ-hydroxyméthyl-9-oxo (11*H*)-indolizino-[1,2-*b*] quinoléine-7-yl) propionate de *tert*-butyle (1,45 g, 3,32 mmol) est dissous dans du dichlorométhane anhydre (25 ml) et traité avec une solution saturée de chlorure d'hydrogène dans le dichlorométhane (100 ml). Le mélange résultant est maintenu à -20° C pendant 16 heures. Le précipité est filtré, lavé avec du méthanol et séché sous pression réduite, ce qui permet d'obtenir 662 mg (55 %) du composé recherché sous forme d'un solide jaune, p.f. > 300° C.
RMN-¹H (DMSO) : 0,90 (t, 3H) ; 1,20 (q, 2H) ; 3,27 (dd, 2H) ; 5,29 (s, 2H) ; 5,49 (dd, 2H) ; 7,42 (s, 1H) ; 7,73 (t, 1H) ; 7,90 (t, 1H) ; 8,16 (t, 2H) ; 8,71 (s, 1H).
RMN-C¹³ (DMSO) : 8,45 ; 36,48 ; 42,54 ; 50,68 ; 61,44 ; 73,34 ; 99,78 ; 122,71 ; 127,83 ; 128,15 ; 128,75 ; 129,08 ; 130,07 ; 130,61 ; 131,81 ; 144,66 ; 148,04 ; 152,80 ; 155,91 ; 159,26 ; 172,08.
IR (KBr) : 761 ; 1127 ; 1204 ; 1285 ; 1580 ; 1653 ; 1757.

### Exemple 4 : Acide β-éthyl-β-hydroxy-γ-(8-hydroxyméthyl-9-oxo (11H)-indolizino-[1,2-b] quinoléine-7-yl)-propionique

On ajoute une solution aqueuse d'hydroxyde de potassium (0,1*N*, 30 ml) à la 5-éthyl-4,5-dihydro-5-hydroxy-1*H*-oxepino [3',4':6,7]-indolizino [1,2-*b*] quinoléine-3,15 (4*H*,13*H*)-dione (500 mg, 1,38 mmol) et la suspension résultante est agitée à la température ambiante pendant 16 heures, ce qui donne une solution presque limpide qui est filtrée. Le filtrat est acidifié à pH 3,5 avec de l'acide chlorhydrique 1*N*, et le précipité jaune est récupéré par filtration, lavé avec de l'eau et de l'acétone puis séché sous pression réduite. On obtient 415 mg (79 %) du composé recherché sous forme de monohydrate, p.f. 165-167° C.
RMN-¹H (DMSO) : 0,82 (t, 3H) ; 2,10 (m, 2H) ; 2,83 (d, 2H) ; 3,12 (d, 2H) ; 3,25 (se, 1H) ; 4,81 (s, 2H) ; 5,26 (s, 2H) ; 5,76 (se, 1H) ; 7,38 (s, 1H) ; 7,71 (t, 1H) ; 7,84 (t, 1H) ; 8,10 (d, 1H) ; 8,18 (d, 1H) ; 8,34 (s, 1H) ; 12,15 (se, 1H).
RMN-C¹³ (DMSO) : 8,16 ; 34,80 ; 46,71 ; 50,36 ; 55,73 ; 76,53 ; 100,17 ; 127,50 ; 128,00 ; 128,26 ; 128,69 ; 129,06 ; 130,01 ; 130,45 ; 131,63 ; 142,57 ; 148,09 ; 153,19 ; 156,07 ; 161,22 ; 172,27.
IR (KBr) : 1020 ; 1188 ; 1413 ; 1586 ; 1651 ; 1694.

### Exemple 5 : β-éthyl-β-hydroxy-γ-(8-hydroxyméthyl-9-oxo (11H)-indolizino-[1,2-b] quinoléine-7-yl)-propionate de méthyle

De la 5-éthyl-4,5-dihydro-5-hydroxy-1*H*-oxepino [3',4':6,7]-indolizino [1,2-*b*] quinoléine-3,15 (4*H*,13*H*)-dione (180 mg, 0,5 mmol), en suspension dans le méthanol (50 ml) est traitée avec du chlorure d'hydrogène sec 6*N* dans le méthanol (0,5 ml) et maintenue au reflux jusqu'à dissolution complète (4 heures). Les composés volatils sont évaporés et le résidu est dissous dans du dichlorométhane (50 ml), lavé avec de l'hydroxyde de sodium dilué (0,05 *N*, 15 ml) et de la saumure (15 ml). La fraction organique est séchée sur sulfate de sodium et évaporée. Le solide résiduel est purifié par chromatographie sur colonne de gel de silice (MeOH à 3 % / CH₂Cl₂) et le produit purifié est repris dans du diéthyl éther, filtré et séché, ce qui donne 120 mg (58 %) du composé recherché sous forme d'un solide jaune pâle, p.f. 163-166° C.
RMN-¹H (CDCl₃) : 0,93 (t, 3H) ; 2,2 (m, 2H) ; 3,05 (dd, 2H) ; 3,49 (s, 3H) ; 3,62 (s, 3H) ; 4,93 (s, 2H) ; 5,22 (d, 2H) ; 5,52 (s, 1H) ; 7,21 (s, 1H) ; 7,62 (t, 1H) ; 7,81 (t, 1H) ; 7,91 (d, 1H) ; 8,22 (d, 1H) ; 8,36 (s, 1H).
RMN-C¹³ (CDCl₃) : 7,74 ; 35,54 ; 46,82 ; 50,15 ; 51,67 ; 58,10 ; 65,33 ; 78,03 ; 100,17 ; 125,57 ; 127,70 ; 128,04 ; 128,10 ; 128,35 ; 129,53 ; 130,39 ; 130,94 ; 143,87 ; 148,75 ; 152,94 ; 157,83 ; 161,74 ; 171,35.
IR (KBr) : 1207 ; 1595 ; 2655 ; 1709.

### Exemple 6 : β-éthyl-α,α-difluoro-β-hydroxy-γ-(8-hydroxyméthyl-9-oxo (11H)-indolizino-[1,2-b] quinoléine-7-yl)-propionate d'éthyle

On ajoute, sous argon, goutte à goutte, à une suspension de zinc (1,25 g, 17,2 mmol) dans du THF anhydre au reflux (40 ml) à peu près la moitié d'une quantité totale de bromodifluoroacétate d'éthyle (1,8 ml, 14 mmol), de la 8-formyloxyméthyl-7-propionylindolizino [1,2-*b*] quinoléine-9 (11*H*)-one (2,0 g, 5,75 mmol, telle qu'obtenue dans l'exemple 1.b.) en suspension dans du THF anhydre (10 ml), puis la partie restante de bromodifluoroacétate d'éthyle. Le mélange réactionnel est maintenu au reflux pendant encore une demi-heure. Après refroidissement à la température ambiante, la réaction est arrêtée par ajout de chlorure d'ammonium aqueux saturé (20 ml) et le mélange réactionnel extrait avec du dichlorométhane (3 x 20 ml). Les extraits organiques combinés sont séchés et concentrés. Le résidu est repris dans du diéthyl éther (10 ml), filtré et purifié par chromatographie sur colonne (SiO₂, CH₂Cl₂ MeOH:98/2), ce qui donne 664 mg (26 %) de produit sous forme d'un solide jaune, p.f. 208-209° C.
RMN-¹H (CDCl₃) : 0,91 (t, 3H) ; 1,38 (t, 3H) ; 2,32 (m, 2H) ; 4,8 (se, 1H) ; 4,38 (q, 2H) ; 5,09 (d, 2H) ; 5,13 (dd, 2H) ; 7,42 (s,1H) ; 7,55 (t, 1H) ; 7,72 (t, 1H) ; 7,79 (d, 1H) ; 8,08 (d, 1H) ; 8,22 (s, 1H)
RMN-C¹³ (CDCl₃) : 6,97 ; 13,93 ; 28,63 ; 50,18 ; 56,27 ; 63,15 ; 77,20 ; 81,96 (t) ; 101,27 ; 116,40(t) ; 127,67 ; 127,77 ; 127,97 ; 128,31 ; 129,26 ; 130,33 ; 130,94 ; 131,23 ; 143,16 ; 148,34 ; 150,20 ; 151,91 ; 161,21 ; 163,21 (t).
IR (KBr) : 1124 ; 1308 ; 1591 ; 1647 ; 1748.

### Exemple 7 : β-éthyl-β-hydroxy-γ-(8-hydroxyméthyl-9-oxo (11H)-indolizino-[1,2-b] quinoléine-7-yl)-propionate d'éthyle

Une suspension de zinc (1,25 g, 19,1 mmol), de la 8-méthyl-7-propionylindolizino [1,2-*b*]quinoléine-9-(11*H*)-one (500 mg, 1,43 mmol, telle qu'obtenue par Kingsburry, W. D., Tetrahedron Lett. 29:6847 (1988)) et de l'acétate d'argent (250 mg, 1,50 mmol) dans du tétrahydrofuranne anhydre (10 ml) sont agités à la température ambiante sous atmosphère d'argon. Au bout de 10 minutes, le mélange réactionnel est activé par addition, goutte à goutte, d'une solution molaire de chlorodiéthylaluminium (10 ml, 10 mmol), puis du bromoacétate d'éthyle (1,25 ml, 11,3 mmol) est ajouté goutte à goutte et le mélange résultant est laissé à réagir pendant encore 5 heures. La réaction est arrêtée par addition successive d'alcool éthylique (10 ml) et d'une solution saturée de tartrate de potassium et de sodium (10 ml). Le mélange résultant est agité encore une heure, filtré et concentré sous pression réduite. Le résidu est repris dans du dichlorométhane (30 ml), lavé avec de l'eau, séché, concentré et purifié par chromatographie sur colonne (SiO₂, CH₂Cl₂ / MeOH:98/2), ce qui donne 93 mg (15 %) de produit souhaité sous forme d'un solide jaune pâle, p.f. 185-188° C.
RMN-¹H (CDCl₃) : 0,91 (t, 3H) ; 1,17 (t, 3H) ; 1,99 (m, 2H) ; 2,49 (s, 3H) ; 3,10 (dd, 2H) ; 4,11 (q, 2H) ; 4,6 (se, 1H) ; 5,25 (s, 2H) ; 7,65 (t, 1H) ; 7,67 (s,1H) ; 7,80 (t, 1H) ; 7,90 (d, 1H) ; 8,22 (d, 1H) ; 8,34 (s, 1H).
RMN-C¹³ (CDCl₃) : 8,02 ; 13,99 ; 14,72 ; 33,14 ; 43,97 ; 50,02 ; 61,0 ; 76,54 ; 101,90 ; 127,65 ; 127,84 ; 128,08 ; 128,81 ; 128,88 ; 130,74 ; 131,59 ; 131,65 ; 140,33 ; 147,64 ; 152,96 ; 153,61 ; 162,11 ; 172,91.
IR (KBr) : 762 ; 1192 ; 1576 ; 1653 ; 1740.

### Exemple 8 : β-éthyl-β-hydroxy-γ-(8-hydroxyméthyl-9-oxo (11H)-indolizino-[1,2-b] quinoléine-7-yl)-propionate de tert-butyle

On ajoute goutte à goutte de l'anhydride acétique (70 µl, 0,7 mmol) à une solution de β'-éthyl-β-hydroxy-γ-(8-hydroxyméthyl-9-oxo (11*H*)-indolizino-[1,2*-b*] quinoléine-7-yl)-propionate de *tert*-butyle (200 mg, 0,46 mmol) et de triéthylamine (140 µl, 1 mmol) dans du dichlorométhane (5 ml) et le mélange résultant est agité à la température ambiante pendant 21 heures. Les composants volatils sont évaporés et le résidu est purifié par chromatographie sur colonne de gel de silice (1-2 % MeOH / CH₂Cl₂), ce qui donne 152 mg de composé recherché sous forme d'un solide jaune, p.f. 195-196° C.
RMN-¹H (CDCl₃) : 0,88 (t, 3H) ; 1,32 (s, 9H) ; 1,93 (m, 2H) ; 2,07 (s, 3H) ; 2,97 (dd, 2H) ; 4,8 (se, 1H) ; 5,28 (s, 2H) ; 5,59 (dd, 2H) ; 7,39 (s, 1H) ; 7,63 (t, 1H) ; 7,80 (t, 1H) ; 7,90 (d, 1H) ; 8,23 (d, 1H) ; 8,34 (s, 1H).
RMN-C¹³ (CDCl₃) : 8,02 ; 21,06 ; 27,91 ; 35,05 ; 45,58 ; 50,16 ; 59,23 ; 77,52 ; 82,26 ; 100,59 ; 124,21 ; 127,91 ; 128,10 ; 128,14 ; 128,97 ; 129,18 ; 130,68 ; 131,46 ; 142,85 ; 148,29 ; 152,43 ; 158,49 ; 161,83 ; 171,13 ; 171,90.

### Exemple 9 : 5,12-diéthyl-4,5-dihydro-5-hydroxy-1H-oxepino [3',4':6,7]-indolizino [1,2-b] quinoléine-3,15 (4H,13H)-dione

Ce composé est préparé d'une manière analogue à l'exemple 1, excepté que dans l'étape 1.a., la 7-éthyl camptothécine (Sawada et ses collaborateurs, Chem. Pharm. Bull. 39:2574 (1991)) est utilisée à la place de la camptothécine. On obtient le composé recherché sous la forme d'un solide jaune vif, p.f. > 270° C.
RMN-¹H (DMSO) : 0,92 (t, 3H) ; 1,39 (t, 3H) ; 1,93 (q, 2H) ; 3,08 (d, 2H) ; 3,25 (q, 2H) ; 3,51 (d, 2H) ; 5,32 (s, 2H) ; 5,52 (dd, 2H) ; 7,42 (s, 1H) ; 7,76 (t, 1H) ; 7,89 (t, 1H) ; 8,18 (d,1H) ; 8,32 (d,1H).
RMN-C¹³ (DMSO) : 8,46 ; 14,15 ; 22,42 ; 36,50 ; 42,54 ; 49,95 ; 61,45 ; 73,35 ; 99,68 ; 122,61 ; 124,27 ; 126,76 ; 127,70 ; 128,27 ; 129,92 ; 130,18 ; 145,17 ; 145,82 ; 148,57 ; 152,15 ; 155,89 ; 159,26 ; 172,08.

### Exemple 10 : Acide β-éthyl-γ-(12-éthyl-8-hydroxyméthyl-9-oxo (11H)-indolizino-[1,2-b] quinoléine-7-yl)-β-hydroxy-propionique

Ce composé est préparé d'une manière analogue à l'exemple 4, excepté que la 5,12-diéthyl-4,5-dihydro-5-hydroxy-1*H*-oxepino [3',4':6,7]-indolizino [1,2-*b*] quinoléine-3,15 (4*H*,13*H*)-dione est utilisée à la place de la 5-éthyl-4,5-dihydro-5-hydroxy-1*H*-oxepino [3',4':6,7]-indolizino [1,2-*b*] quinoléine-3,15 (4*H*,13*H*)-dione. Elle se présente sous la forme d'un solide d'un blanc légèrement sale, p.f. 238-239° C.
RMN-¹H (DMSO) : 0,82 (t, 3H) ; 1,35 (t, 3H) ; 2,01 (m, 2H) ; 2,85 (d, 2H) ; 3,18 (d, 2H) ; 3,22 (q, 2H) ; 4,81 (s, 2H) ; 5,00 (se, 1H) ; 5,24 (s, 2H) ; 5,78 (se, 1H) ; 7,38 (s, 1H) ; 7,77 (t, 1H) ; 7,86 (t, 1H) ; 8,18 (d, 1H) ; 8,28 (d, 1H) ; 12,10 (se, 1H).
RMN-C¹³ (DMSO) : 8,12 ; 14,15 ; 22,41 ; 34,78 ; 46,74 ; 49,65 ; 55,71; 76,51; 100,04 ; 124,22 ; 126,63 ; 127,48 ; 128,12 ; 128,21 ; 129,94 ; 130,02 ; 143,10 ; 145,59 ; 148,69 ; 152,62 ; 156,03 ; 161,22 ; 172,22.

### Exemple 11 : 8-éthyl-2,3,8,9-tétrahydro-8-hydroxy-10H,12H-[1,4] dioxino [2,3-g] oxepino [3',4':6,7] indolizino [1,2-b] quinoléine-10,13 (15H)-dione

### 11.a. 2-éthyl-2-(2-méthoxy-4-pyridyl)-1,3-dioxolane (F)

On distille de l'eau de manière azéotropique (une nuit entière) avec un appareil Dean Stark à partir d'un mélange de 2-chloro-4-propionylpyridine (10 g, 59 mmol) obtenue comme dans Lamattina, J.L. *J*. *Hétérocyclic Chem*. *20,* p. 553 (1983)), d'éthylène glycol (20 ml) et d'acide *p*-toluènesulfonique (250 mg) dans le toluène (150 ml). Le solvant est ensuite éliminé sous pression réduite, l'acide est neutralisé avec du bicarbonate de sodium aqueux saturé (100 ml) et le produit est extrait à l'éther. Les extraits éthérés combinés sont lavés avec de la saumure, séchés sur sulfate de sodium et évaporés, ce qui donne 13,3 g (96 %) de produit brut protégé par le groupe carbonyle qui est porté au reflux avec 3 équivalents de méthoxyde de sodium dans l'acétonitrile jusqu'à la fin de la réaction (contrôle par chromatographie sur couche mince : SiO₂, oxyde de *tert*-butyle méthyle / hexane (TBMO/HX) 50/50). La solution d'acétonitrile est ensuite filtrée et évaporée. Le résidu est repris dans l'éther, lavé avec de l'eau et de la saumure, séché sur sulfate de sodium et évaporé, ce qui donne une huile marron qui est distillée (70-75° C, 0,04 mbar) ; on récupère 10,7 g (rendement global 81 %) de produit (F) sous forme d'une huile limpide.

### 11.b. 2-éthyl-2-(3-hydroxyméthyl-2-méthoxy-4-pyridyl)-1,3-dioxolane (G)

On ajoute goutte à goutte au moyen d'une canule du *tert*-butyllithium (1,7 *M* dans le pentane, 100 ml, 170 mmol) à une solution de bromomésitylène (13 ml, 85 mmol) dans du tétrahydrofurane anhydre (300 ml) à -78° C et sous argon. Le précipité blanc résultant est agité à -78° C pendant une heure puis du 2-éthyl-2-(2-méthoxy-4-pyridyl)-1,3-dioxolane (10 g, 44,8 mmol) est ajouté et le mélange réactionnel est agité 15 minutes à -78° C, une heure à 0° C et une heure à la température ambiante. Après un nouveau refroidissement à -78° C, de la N,N-diméthylformamide anhydre (100 mmol) est ajoutée et le mélange réactionnel est laissé se réchauffer jusqu'à la température ambiante puis est agité pendant 16 heures, après quoi une analyse par chromatographie sur couche mince (SiO₂, TBMO/HX:50/50) fait apparaître la consommation complète du produit de départ. La réaction est arrêtée avec du chlorure d'ammonium saturé et le mélange réactionnel extrait avec du diéthyl éther (200 ml, 50 ml, 50 ml). Les extraits combinés sont séchés sur sulfate de sodium et évaporés, ce qui donne une huile jaune qui est purifiée par chromatographie sur colonne (SiO₂, TBMO/HX: 0/100 à 5/95 pour éluer les dérivés mésytylène puis 20/80 à 50/50 pour éluer le produit) pour obtenir l'aldéhyde intermédiaire (7 g). L'aldéhyde est dissous dans le méthanol (100 ml) et traité avec du borohydrure de sodium (5 g, 132 mmol) et le mélange résultant est agité jusqu'à consommation complète de l'aldéhyde intermédiaire (environ 1 heure) avec contrôle analytique par chromatographie sur couche mince. Le solvant est ensuite évaporé, le résidu est repris dans l'éther, lavé avec de l'eau et de la saumure, séché, et le solvant est évaporé. La chromatographie sur colonne (SiO₂, TBMO/HX: 10/90 à 50/50) du résidu donne 7 g (rendement global 62 %) de produit (G) sous forme d'une huile jaune.

### 11.c. 2-(3-benzyloxyméthyl-2-méthoxy-4-pyridyl)-2-éthyl-1,3-dioxolane (H)

On ajoute goutte à goutte une solution de 2-éthyl-2-(3-hydroxyméthyl-2-méthoxy-4-pyridyl)-1,3-dioxolane (7 g, 30 mmol) et de chlorure de benzyle (5 ml, 45 mmol) dans du tétrahydrofurane anhydre (50 ml) à une suspension d'hydrure de sodium (80 % dans l'huile minérale, 1,85 g, 61 mmol) dans du tétrahydrofurane anhydre (100 ml) et on maintient au reflux le mélange réactionnel pendant 16 heures. Le mélange réactionnel est ensuite laissé à refroidir jusqu'à la température ambiante, la réaction est arrêtée avec de l'eau (50 ml) et le mélange réactionnel concentré sous pression réduite. Le résidu est dissous dans du diéthyl éther (150 ml) et lavé avec de l'eau et de la saumure, séché et évaporé. Une purification par chromatographie sur colonne (SiO₂, TBMO/HX: 5/95 à 20/80) donne le produit protégé par le benzyle (H), 9 g, (87 %) sous forme d'une huile limpide.

### 11.d. 1-(3-benzyloxyméthyl-2-méthoxy-4-pyridyl)-propane-1-one (I')

On traite du 2-(3-benzyloxyméthyl-2-méthoxy-4-pyridyl)-2-éthyl-1,3-dioxolane (9 g, 27 mmol) avec de l'acide trifluoracétique (10 ml) et de l'eau (5 ml) à une température de bain de 120° C pendant 3 heures. Le mélange réactionnel est concentré sous pression réduite et les traces résiduelles d'acides sont neutralisées par l'addition de bicarbonate de sodium aqueux saturé. L'extraction avec de l'éther suivie d'une chromatographie sur colonne (SiO₂, TBMO/HX: 10/90) donne 5,5 g (70 %) de produit (I).

### 11.e. β-éthyl-β-hydroxy-γ-(3-benzyloxyméthyl-2-méthoxy-4-pyridyl) propionate de tert-butyle (J)

On ajoute goutte à goutte du bromoacétate de *tert*-butyle (13 ml, 80 mmol) à une suspension de zinc (5,3 g, 80 mmol activée par traitement avec HCl 6 *N* en 10 secondes, puis lavée successivement avec de l'eau jusqu'à pH neutre, de l'acétone et du diéthyl éther) dans le tétrahydrofurane anhydre (60 ml) au reflux. Le milieu réactionnel est maintenu au reflux pendant encore 10 minutes après que l'addition soit terminée. Ensuite, on ajoute une solution de 1-(3-benzyloxyméthyl-2-méthoxy-4-pyridyl)-propane-1-one (5,8 g, 20 mmol) dans le tétrahydrofurane anhydre (20 ml) et on agite le mélange réactionnel sous reflux pendant une heure supplémentaire. La réaction est arrêtée à 0° C avec du chlorure d'ammonium aqueux saturé (100 ml) et le mélange réactionnel extrait avec du diéthyl éther. Les extraits combinés sont séchés sur sulfate de sodium et évaporés, ce qui donne une huile jaune qui est purifiée par chromatographie sur colonne (SiO₂, TBMO/HX: 5/95 à 10/90) pour obtenir l'ester de *tert*-butyle (J) (7 g, 95 %) sous forme d'une huile limpide.

### 11.f. β-éthyl-β-hydroxy-γ-(3-hydroxyméthyl-2-méthoxy-4-pyridyl) propionate de tert-butyle (K)

On hydrogénolyse du β-éthyl-β-hydroxy-γ-(3-benzyloxyméthyl-2-méthoxy-4-pyridyl) propionate de *tert*-butyle (1 g, 2,5 mmol) à la pression atmosphérique et à la température ambiante en utilisant 5 % de palladium sur charbon comme catalyseur (50 mg) et de l'éthanol absolu comme solvant (10 ml). Une fois la réaction terminée (6 heures), le catalyseur est séparé par filtration et le solvant est évaporé, ce qui laisse 0,7 g (90 %) de produit (K) d'une pureté suffisante pour une utilisation synthétique ultérieure.

### 11.g. 5-éthyl-1,5-dihydro-5-hydroxy-9-méthoxy-oxepino [3,4-c] pyridine-3 (4H)-one (L)

Du β-éthyl-β-hydroxy-γ-(3-hydroxyméthyl-2-méthoxy-4-pyridyl) propionate de *tert*-butyle (8,8 g, 28 mmol) est traité avec de l'acide trifluoracétique (30 ml) pendant 3 heures à la température ambiante. Les composants volatils sont évaporés et le résidu est purifié par chromatographie sur colonne (SiO₂, CH₂Cl₂/MeOH: 100/0 à 98/2), ce qui donne une huile limpide qui, après traitement avec du toluène, donne 5,9 g de produit (L) (89 %) sous forme de cristaux blancs, p.f. 97-98° C.

### 11.h. 5-éthyl-1,5-dihydro-5-hydroxy-oxepino [3,4-c] pyridine 3,9(4H,8H)-dione (M)

On chauffe au reflux pendant 9 heures dans de l'acide chlorhydrique 1 *N* (20 ml) de la 5-éthyl-1,5-dihydro-5-hydroxy-9-méthoxy-oxepino [3,4-c] pyridine-3 (4*H*)-one (0,5 g, 2,1 mmol). Le mélange réactionnel est concentré sous pression réduite et le résidu est encore séché par addition et évaporation du toluène, par deux fois, puis laissé une nuit entière sous pression réduite en présence de pentoxyde de phosphore. L'huile résultante est dissoute dans l'acétonitrile anhydre (5 ml) et agitée sous argon pendant 24 heures. Le précipité est filtré et séché, ce qui donne 0,23 g (49 %) d'un solide blanc (M), p.f. 118-119° C.

### 11.i. 6,7-éthylènedioxy-2-iodo-3-quinoléine-méthanol (N)

Les procédures décrites par Meth-Cohn et ses collaborateurs, J. Chem. Soc. Perkin Trans. I, p. 1520 (1981) ; Meth -Cohn, J. Chem. Soc. Perkin Trans I, p. 2509 (1981) ; et Nakasimhan et ses collaborateurs, J. Am. Chem. Soc. *112*, p. 4431 (1990), sont employées. De la 3,4-éthylènedioxyacétanilide (22 g, 113 mmol) est ajoutée au réactif de Vilsmeyer obtenu par addition goutte à goutte d'oxychlorure de phosphoryle (71 ml, 0,77 mol) à de la diméthylformamide anhydre (23 ml, 0,28 mol), refroidie avec un bain eau / glace et agitée pendant encore 0,5 heure sous atmosphère d'argon. Le mélange résultant est chauffé à 75° C pendant 16 heures. Après refroidissement à la température ambiante, le mélange réactionnel est ajouté à un mélange de glace et d'eau (300 ml) et extrait avec du dichlorométhane (5 x 200 ml). Les extraits organiques combinés sont séchés sur sulfate de sodium, filtrés et concentrés. Le résidu solide est mis en suspension dans du dichlorométhane (20 ml), filtré et séché sous pression réduite, ce qui donne 10 g (35 %) de 2-chloro-6,7-éthylènedioxyquinoléine-3-carbaldéhyde sous forme d'un solide jaune, p.f. 222-224° C. Cet intermédiaire est traité avec de l'iodure de sodium (30 g, 0,2 mol) et de l'acide chlorhydrique concentré (1,5 ml) dans de l'acétonitrile au reflux (150 ml) pendant 24 heures. Après refroidissement à la température ambiante, le solvant est éliminé sous pression réduite et le résidu est repris dans du tétrahydrofuranne aqueux à 50 % (200 ml), filtré, lavé avec du tétrahydrofuranne et séché sous pression réduite, ce qui donne 12 g de 6,7-éthylènedioxy-2-iodoquinoléine-3-carbaldéhyde sous forme d'un solide jaune, p.f. 155-157° C. L'intermédiaire ci-dessus est traité avec du borohydrure de sodium (2 g, 52 mmol) dans le méthanol (200 ml) à la température ambiante pendant 0,5 heure. Le solvant est éliminé sous pression réduite et le résidu est repris dans l'eau et filtré. Le solide résultant est séché sous pression réduite en présence de pentoxyde de phosphore, ce qui donne 11 g de (6,7-éthylènedioxy-2-iodoquinoléine-3-yl)-méthanol sous forme d'un solide jaune, p.f. 178-180° C.

### 11.j. 5-éthyl-8-(6,7-éthylènedioxy-2-iodo-3-quinoléineméthyl)-1,5-dihydro-5-hydroxy-oxepino [3,4-c] pyridine 3,9(4H,8H)-dione (O)

On ajoute goutte à goutte pendant 5 minutes de l'azodicarboxylate de diéthyle (570 µl, 3,6 mmol) à une solution de 5-éthyl-1,5-dihydro-5-hydroxy-oxepino [3,4-c] pyridine 3,9(4*H*,8*H*)-dione (400 mg, 1,79 mmol), du composé obtenu à l'étape précédente 11.i. (770 mg, 2,23 mmol) et de triphénylphosphine (934 mg, 3,58 mmol) dans un mélange THF/DMSO anhydre (8/1 v/v, 45 ml) et on agite le mélange résultant sous argon à la température ambiante pendant 16 heures. Le mélange réactionnel est ensuite concentré sous pression réduite et le résidu est dissous dans du chloroforme (100 ml). La solution résultante est lavée avec de la saumure (4 x 50 ml), séchée sur sulfate de sodium et évaporée. Le résidu est purifié par chromatographie sur colonne (SiO₂, CH₂Cl₂/MeOH: 99/1 à 98/2), ce qui donne 650 mg (66 %) du produit (O) sous forme d'un solide blanc, p.f. 165-167° C.

### 11.k. 8-éthyl-2,3,8,9-tétrahydro-8-hydroxy-10H,12H-[1,4] dioxino [2,3-g] oxepino [3',4':6,7] indolizino [1,2-b] quinoléine-10,13 (15H)-dione

De la 5-éthyl-8-(6,7-éthylènedioxy-2-iodoquinoléine-3-yl)méthyl-4,5-dihydro-5-hydroxy-(1*H*, 3*H*)oxepino [3,4-c] pyridine-3-dione (600 mg, 1,1 mmol), du bromure de tétrabutyl-ammonium (352 mg, 1,1 mmol), de l'acétate de sodium (359 mg, 4,4 mmol) et de l'acétate de palladium II (98 mg, 0,43 mmol) sont dissous dans de l'acétonitrile anhydre (40 ml) et chauffés à 90° C sous argon pendant 16 heures. Après refroidissement à la température ambiante, un précipité blanc est séparé de la solution rougeâtre. Ce précipité est filtré et séché sous pression réduite. Le produit brut est mis en suspension dans l'eau, filtré et séché sous pression réduite sur du pentoxyde de phosphore, ce qui donne 250 mg du composé recherché sous forme d'un solide jaune clair, p.f. > 250° C.
RMN-¹H (DMSO) : 0,91 (t, 3H) ; 1,87 (m, 2H) ; 3,08 (d, 1H) ; 3,51 (d, 1H) ; 4,45 (s, 4H) ; 5,19 (s, 2H) ; 5,47 (dd, 2H) ; 6,02 (se, 1H) ; 7,33 (s, 1H) ; 7,54 (s,1H) ; 7,55 (s, 1H) ; 8,43 (s, 1H).
RMN-C¹³ (DMSO) : 8,43 ; 36,47 ; 42,54 ; 50,52 ; 61,43 ; 64,43 (2C) ; 73,31 ; 99,07 ; 112,27 ; 113,14 ; 122,00 ; 124,24 ; 128,18 ; 129,74 ; 144,59 ; 145,01 ; 145,33 ; 147,63 ; 150,88 ; 155,88 ; 159,23 ; 172,07.

### Exemple 12 : 10-benzyloxy-5-éthyl-4,5-dihydro-5-hydroxy-1H-oxepino [3',4':6,7]-indolizino [1,2-b]quinoléine-3,15 (4H,13H)-dione

### 12.a. (6-benzyloxy-2-iodo-3-quinoléine)-méthanol

Ce composé est préparé d'une manière analogue à celle indiquée dans l'étape 11.i. de l'exemple 11, mais en utilisant de la 4-benzyloxyacétanilide à la place de la 3,4-éthylènedioxyacétanilide. Une purification par chromatographie sur colonne de gel de silice et l'utilisation de dichlorométhane comme éluant sont nécessaires pour isoler (rendement 8 %) à une pureté suffisante, l'intermédiaire 6-benzyloxy-2-chloroquinoléine-3-carbaldéhyde, p.f. 180-182° C. Ensuite, l'échange d'halogène donne le 6-benzyloxy-2-iodoquinoléine-3-carbaldéhyde, p.f. 155-157° C et une réduction subséquente au borohydrure de sodium donne le (6-benzyloxy-2-iodoquinoléine-3-yl)-méthanol, p.f. 147-149° C.

### 12.b. 8-(6-benzyloxy-2-iodo-3-quinoléineméthyl)-1,5-dihydroxy-5-éthyl-5-hydroxy-oxepino [3,4-c] pyridine-3,9(4H,8H)-dione

Ce composé est préparé d'une manière analogue à celle indiquée dans l'étape 11.j. de l'exemple 11, mais en utilisant du (6-benzyloxy-2-iodoquinoléine-3-yl)-méthanol à la place du (6,7-éthylènedioxy-2-iodoquinoléine-3-yl)-méthanol. Ce composé se présente sous la forme d'un solide blanc, p.f. 197-199° C.

### 12.c. 10-benzyloxy-5-éthyl-4,5-dihydro-5-hydroxy-1H-oxepino [3',4':6,7]-indolizino [1,2-b]quinoléine-3,15 (4H,13H)-dione

Ce composé est préparé d'une manière analogue à celle indiquée dans l'étape 11.k. de l'exemple 11, mais en utilisant la 8-(6-benzyloxy-2-iodo-3-quinoléineméthyl)-1,5-dihydroxy-5-éthyl-5-hydroxy-oxepino [3,4-c] pyridine-3,9(4*H*, 8*H*)-dione à la place de la 5-éthyl-8-(6,7-éthylènedioxy-2-iodoquinoléine-3-yl)méthyl-4,5-dihydro-5-hydroxy-(1*H*, 3*H*)oxepino [3,4-c] pyridine-3-dione. Le composé recherché se présente sous forme d'un solide jaune clair, p.f. > 250° C.
RMN-¹H (DMSO) : 0,90 (t, 3H) ; 1,85 (m, 2H) ; 3,08 (d, 1H) ; 3,50 (d, 1H) ; 5,25 (s, 2H); 5,30 (s, 2H); 5,50 (dd, 2H) ; 6,05 (s, 1H) ; 7,30-7,70 (m, 8H) ; 8,10 (d, 1H) ; 8,55 (s, 1H).
RMN-C¹³ (DMSO): 8,43 ; 36,48 ; 38,28 ; 50,65 ; 61,42 ; 70,00 ; 73,32 ; 99,05 ; 107,71 ; 122,05 ; 123,42 ; 128,18 ; 128,26 ; 128,70 ; 129,40 ; 130,19 ; 130,48 ; 130,63 ; 136,65 ; 144,18 ; 144,90 ; 150,53 ; 155,91 ; 157,31 ; 159,24 ; 172,06.

### Exemple 13 : Acide γ-(12-benzyloxy-8-hydroxyméthyl-9-oxo (11H)-indolizino [1,2-b] quinoléine-7-yl)-β-éthyl-β-hydroxy-propionique (E)

Ce composé est préparé d'une manière analogue à celle expliquée dans l'exemple 4, mais en utilisant la 10-benzyloxy-5-éthyl-4,5-dihydro-5-hydroxy-1*H*-oxepino [3',4':6,7]-indolizino [1,2-*b*]quinoléine-3,15 (4*H*,13*H*)-dione à la place de la 5-éthyl-4,5-dihydro-5-hydroxy-1*H*-oxepino [3',4':6,7]-indolizino [1,2-*b*] quinoléine-3,15 (4*H*,13*H*)-dione. Elle se présente sous forme d'un solide jaune, p.f. 171-173° C.
RMN-¹H (DMSO) : 0,80 (t, 3H) ; 2,00 (m, 2H) ; 2,85 (d, 1H) ; 3,15 (d, 1H) ; 4,80 (s, 2H) ; 5,25 (s, 2H) ; 5,30 (s, 2H) ; 5,75 (se, 1H) ; 7,30 (s, 1H) ; 7,35-7,70 (m, 7H) ; 8,10 (d, 1H) ; 8,55 (s, 1H).
RMN-C¹³ (DMSO) : 8,11 ; 34,75 ; 46,68 ; 50,35 ; 55,70 ; 69,97 ; 76,51 ; 99,45 ; 107,78 ; 123,28 ; 127,64 ; 128,18 (2C) ; 128,26 ; 128,70 (2C) ; 129,33 ; 130,17 ; 130,47 ; 130,57 ; 136,69 ; 142,79 ; 144,17 ; 150,93 ; 156,03 ; 157,19 ; 161,20.

### Exemple 14 : 5-éthyl-4,5-dihydro-5,10,-dihydroxy-1H-oxepino [3',4':6,7]-indolizino [1,2-b]quinoléine-3,15 (4H,13H)-dione

La 10-benzyloxy-5-éthyl-4,5-dihydro-5-hydroxy-1*H*-oxepino [3',4':6,7]-indolizino [1,2-*b*]quinoléine-3,15 (4*H*,13*H*)-dione (370 mg, 0,79 mmol) est traitée par hydrogène à la pression atmosphérique et à température ambiante en utilisant du palladium à 10 % sur du charbon comme catalyseur (60 mg) et de l'acide trifluoroacétique comme solvant (15 ml). Une fois la réaction terminée (16 heures), du dichlorométhane (50 ml) et du méthanol (50 ml) sont ajoutés au mélange réactionnel, le catalyseur est filtré et les composants volatils sont évaporés sous pression réduite, ce qui permet d'obtenir le composé recherché brut contenant des traces d'acide trifluoroacétique. Ces traces sont éliminées par co-distillation avec du 1,4-dioxane. On obtient le produit sous forme d'un solide orange, p.f. 150° C (d), d'une pureté suffisante pour une utilisation synthétique ultérieure.
RMN-¹H (DMSO) : 0,89 (t, 3H) ; 1,85 (q, 2H) ; 3,02 (d, 1H); 3,45 (d, 1H) ; 5,19 (s, 2H) ; 5,37 (d, 1H) ; 5,50 (d, 1H) ; 5,98 (se, 1H) ; 7,26 (s 1H) ; 7,31 (s, 1H) ; 7,40 (d, 1H) ; 8,00 (d, 1H) ; 8,42 (s, 1H) ; 10,32 (s, 1H).
RMN-C¹³ (DMSO) : 8,47 ; 36,50 ; 42,61 ; 50,57 ; 61,46 ; 73,35 ; 98,84 ; 109,02 ; 121,83 ; 123,18 ; 129,50 ; 129,85 ; 130,12 ; 130,80 ; 143,39 ; 145,10 ; 149,69 ; 155,97 ; 156,82 ; 159,30 ; 172,11.

### Exemple 15 : 11-(diméthylamino)méthyl-5-éthyl-4,5-dihydro-5,10-dihydroxy-1H-oxepino [3',4':6,7]-indolizino [1,2-b] quinoléine-3,15 (4H,13H)-dione

### 15.a. 11-(diméthylamino)méthyl-5-éthyl-4,5-dihydro-5,10-dihydroxy-1H-oxepino [3',4':6,7]-indolizino [1,2-b]quinoléine-3,15 (4H,13H)-dione

Une suspension de la 10-benzyloxy-5-éthyl-4,5-dihydro-5-hydroxy-1*H*-oxepino [3',4':6,7]-indolizino [1,2-*b*]quinoléine-3,15 (4*H*,13*H*)-dione (260 mg, 0,69 mmol) dans l'acide acétique (15 ml) est traitée avec du formaldéhyde aqueux à 37 % (500 µl) et de la diméthylamine aqueuse à 40 % (500 µl) et le mélange résultant est agité à la température ambiante pendant 16 heures. Le mélange réactionnel est concentré jusqu'à siccité et le résidu est purifié par chromatographie sur colonne (SiO₂, CH₂Cl₂/MeOH: 100/0 à 90/10) suivie d'une cristallisation avec l'acétonitrile, ce qui donne 102 mg du composé recherché.

### 15.b. chlorhydrate de 11-(diméthylamino)méthyl-5-éthyl-4,5-dihydro-5,10-dihydroxy-1H-oxepino[3',4':6,7]-indolizino [1,2-b] quinoléine - 3,15 (4H,13H)-dione

On ajoute goutte à goutte de l'acide chlorhydrique dilué (1*N*) à une suspension de 11-(diméthylamino)méthyl-5-éthyl-4,5-dihydro-5,10-dihydroxy-1*H*-oxepino [3',4':6,7]-indolizino [1,2-*b*] quinoléine-3,15 (4*H*,13*H*)-dione (102 mg) dans l'eau, jusqu'à dissolution complète. L'eau est évaporée sous pression réduite et le résidu est mis en suspension dans l'acétonitrile (5 ml) et filtré, ce qui donne 103 mg du sel recherché, p.f. 248° C (d).
RMN-¹H (DMSO) : 0,88 (t, 3H) ; 1,85 (m, 2H) ; 2,84 (s, 6H) ; 3,08 (d, 1H) ; 3,5 (d, 1H) ; 4,73 (s, 2H) ; 5,25 (s, 2H) ; 5,47 (dd, 2H) ; 7,33 (s, 1H) ; 7,38 (s,1H) ; 7,72 (d, 1H) ; 8,19 (d, 1H) ; 8,99 (s, 1H) ; 9,92 (se, 1H) ; 11,45 (s, 1H).
RMN-C¹³ (DMSO) : 8,46 ; 34,36 ; 42,44 (3C) ; 50,61 (2C) ; 61,42 ; 73,35 ; 99,19 ; 108,63 ; 122,21 ; 122,36 ; 126,86 ; 129,13 ; 130,61 ; 133,09 ; 143,53 ; 144,70 ; 149,76 ; 155,98 ; 157,17 ; 159,27 ; 172,06.

### Exemple 16 : 5-éthyl-9-fluoro-4,5-dihydro-5-hydroxy-10-méthoxy-1H-oxepino[3',4':6,7]indolizino[1,2-b]quinoléine-3,15(4H,13H)-dione

Ce composé est obtenu à partir de la 3-fluoro-4-méthoxyaniline selon la méthode illustrée par les étapes 11i, 11j et 11k de l'exemple 11. Solide jaune, p.f. > 250° C.
RMN-¹H (DMSO) : 0,89 (t, 3H) ; 1,85 (q, 2H) ; 3,08 (d, 1H) ; 3,49 (d, 1H) ; 4,00 (s, 3H) ; 5,25 (s, 2H) ; 5,39 (d, 1H) ; 5,51 (d,1H) ; 6,00 (s, 1H) ; 7,32 (s, 1H) ; 7,72 (d, 1H) ; 7,9 (d,1H) ; 8,58 (s, 1H).
RMN-C¹³ (DMSO) : 8,43 ; 36,48 ; 42,51 ; 50,68 ; 56,60 ; 61,42 ; 73,29 ; 99,25 ; 108,68 ; 113,52 ; 122,23 ; 126,33 ; 129,99 ; 130,30 ; 143,79 ; 144,70 ; 148,42 ; 151,18 ; 153,19 ; 155,81 ; 159,20 ; 172,06.
IR (KBr) : 1259 ; 1503 ; 1602 ; 1737.

### Exemple 17 : 9-chloro-5-éthyl-4,5-dihydro-5-hydroxy-10-méthyl-1H-oxepino[3',4':6,7]indolizino[1,2-b]quinoléine-3,15(4H,13H)-dione

Ce composé est obtenu à partir de la 3-chloro-4-méthoxyaniline selon la méthode illustrée par les étapes 11i, 11j et 11k de l'exemple 11. Solide jaune; p.f. > 250° C.
RMN-¹H (DMSO) : 0,85 (t, 3H) ; 1,85 (q, 2H) ; 2,55 (s, 3H) ; 3,07 (d, 1H) ; 3,45 (d, 1H) ; 5,25 (s, 2H) ; 5,39 (d, 1H) ; 5,51 (d, 1H) ; 6,05 (s, 1H) ; 7,39 (s, 1H) ; 8,10 (s, 1H) ; 8,20 (s, 1H) ; 8,60 (s, 1H).
RMN-C¹³ (DMSO): 8,43 ; 20,20; 36,47; 42,49; 50,67; 61,41 ; 73,28; 99,87; 122,82 ; 126,98 ; 127,99 ; 129,60 ; 130,53 ; 131,08 ; 135,64 ; 136,56 ; 144,39 ; 147,11 ; 153,10 ; 155,85 ; 159,18 ; 172,03.
IR (KBr) : 1208 ; 1479 ; 1606 ; 1656 ; 1724.

### Exemple 18 : 5-éthyl-9,10-difluoro-4,5-dihydro-5-hydroxy-1H-oxepino[3',4':6,7]indolizino[1,2-b]quinoléine-3,15(4H,13H)-dione

Ce composé est obtenu à partir de la 3,4-difluoroaniline selon la méthode illustrée par les étapes 11i, 11j et 11k de l'exemple 11. Solide jaune ; p.f. > 250° C.
RMN-¹H (DMSO) : 0,85 (t, 3H) ; 1,85 (q, 2H) ; 3,07 (d, 1H) ; 3,47 (d, 1H) ; 5,25 (s, 2H) ; 5,39 (d, 1H) ; 5,51 (d, 1H) ; 6,05 (s, 1H) ; 7,39 (s, 1H) ; 8,15 (q, 1H) ; 8,25 (q, 1H) ; 8,68 (s, 1H).
RMN-C¹³ (DMSO) : 8,41 ; 36,45 ; 42,48 ; 50,68 ; 61,40 ; 73,25 ; 99,92 ; 114,44 ; 115,42 ; 115,58 ; 122,96 ; 125,52; 130,56 ; 131,46 ; 144,21 ; 145,25 ; 142,36 ; 153,41 ; 155,85 ; 159,15 ; 172,00.
IR (KBr) : 1266 ; 1512 ; 1581 ; 1618 ; 1751.

### Exemple 19 : 7-éthyl-7,8-dihydro-7-hydroxy-9H,11H-[1,3] dioxolo [4,5-g] oxepino [3',4':6,7] indolizino [1,2-b] quinoléine-9,12[14H]-dione

Ce composé est obtenu à partir de la 3,4-méthylènedioxyaniline selon la méthode illustrée par les étapes 11i, 11j et 11k de l'exemple 11. Solide crème ; p.f. >250° C.
RMN-¹H (DMSO) : 0,85 (t, 3H) ; 1,85 (q, 2H) ; 3,07 (d, 1H) ; 3,45 (d, 1H) ; 5,20 (s, 2H) ; 5,39 (d, 1H) ; 5,51 (d, 1H) ; 6,00 (s, 1H) ; 6,30 (s, 2H) ; 7,30 (s, 1H) ; 7,49 (d, 2H) ; 8,45 (s, 1H).
RMN-C¹³ (DMSO): 8,43 ; 36,49 ; 42,56 ; 50,58 ; 61,42 ; 73,31 ; 98,87 ; 102,75 ; 103,33 ; 104,92 ; 121,76 ; 125,74 ; 128,59 ; 130,33 ; 145,08 ; 146,69 ; 148,78 ; 150,19 ; 151,49 ; 155,90 ; 159,24 ; 172,08.
IR (KBr) : 1248 ; 1459 ; 1606 ;1731.

### Exemple 20 : 9-chloro-5-éthyl-4,5-dihydro-5-hydroxy-10-méthoxy-1H-oxepino [3',4':6,7] indolizino [1,2-b] quinoléine-3,15(4H,13H)-dione

Ce composé est obtenu à partir de la 3-chloro-4-méthoxyaniline selon la méthode illustrée par les étapes 11i, 11j et 11k de l'exemple 11. Solide blanc ; p.f. >250° C.
RMN-¹H (DMSO) : 0,85 (t, 3H) ; 1,85 (q, 2H) ; 3,07 (d, 1H) ; 3,45 (d, 1H) ; 4,01 (s, 3H) ; 5,22 (s, 2H) ; 5,39 (d, 1H) ; 5,51 (d, 1H) ; 6,02 (s, 1H) ; 7,31 (s, 1H) ; 7,68 (s, 1H) ; 8,20 (s, 1H) ; 8,55 (s, 1H).
RMN-C¹³ (DMSO) : 8,22 ; 36,27 ; 42,30 ; 50,48 ; 56,69 ; 61,23 ; 73,08 ; 99,16 ; 107,44 ; 122,16 ; 127,12 ; 128,12 ; 129,25 ; 130,02 ; 130,53 ; 143,29 ; 144,37 ; 151,12 ; 153,29 ; 155,71 ; 158,98 ; 171,84.
IR (KBr) : 1056 ; 1256 ; 1483 ; 1592 ; 1657 ; 1747.

### Exemple 21 : 5-éthyl-4,5-dihydro-5-hydroxy-10-méthoxy-1H-oxepino [3',4':6,7] indolizino [1,2-b] quinoléine-3,15(4H,13H)-dione

Ce composé est obtenu à partir de la 4-méthoxyaniline selon la méthode illustrée par les étapes 11.i., 11.j. et 11.k. de l'exemple 11. Solide jaune ; p.f. > 250° C.
RMN-¹H (DMSO) : 0,85 (t, 3H) ; 1,85 (q, 2H) ; 3,07 (d, 1H) ; 3,45 (d, 1H) ; 3,95 (s, 3H) ; 5,28 (s, 2H) ; 5,40 (d, 1H) ; 5,51 (d, 1H) ; 6,00 (s, 1H) ; 7,38 (s, 1H) ; 7,51 (d, 2H) ; 8,07 (d, 1H) ; 8,55 (s, 1H).
RMN-C¹³ (DMSO) : 8,45 ; 36,48 ; 42,51 ; 50,64 ; 55,92 ; 61,42 ; 73,33 ; 99,01 ; 106,49 ; 122,02 ; 123,19 ; 129,59 ; 130,20 ; 130,43 ; 144,17 ; 144,94 ; 150,40 ; 155,92 ; 158,31 ; 159,26; 172,07.
IR (KBr) : 1251 ; 1604 ; 1655 ; 1735.

### Exemple 22 : 9,11-dichloro-5-éthyl-4,5-dihydro-5-hydroxy-1H-oxepino[3',4':6,7]indolizino[1,2-b]quinoléine-3,15(4H,13H)-dione

Ce composé est obtenu à partir de la 3,5-dichloroaniline selon la méthode illustrée par les étapes 11.i., 11.j. et 11.k. de l'exemple 11. Solide jaune ; p.f. > 250° C.
RMN-¹H (DMSO) : 0,85 (t, 3H) ; 1,85 (q, 2H) ; 3,07 (d, 1H) ; 3,45 (d, 1H) ; 5,30 (s, 2H); 5,41 (d, 1H) ; 5,55 (d, 1H) ; 6,08 (s, 1H) ; 7,41 (s, 1H) ; 8,05 (s, 1H) ; 8,21 (s, 1H) ; 8,91 (s, 1H).
RMN-C¹³ (DMSO) : 8,39 ; 36,45 ; 42,51 ; 51,03 ; 61,39 ; 73,25 ; 100,62 ; 123,55 ; 124,63 ; 127,60 ; 128,08 ; 128,56 ; 132,06 ; 132,19 ; 134,53 ; 143,77 ; 148,80 ; 154,88 ; 155,82 ; 159,13 ; 171,98.
IR (KBr) : 1064 ; 1275 ; 1586 ; 1651 ; 1743.

### Exemple 23 : 5-éthyl-9-fluoro-4,5-dihydro-5-hydroxy-10-méthyl-1H-oxepino [3',4':6,7] indolizino [1,2-b]quinoléine-3,15(4H,13H)-dione

Ce composé est obtenu à partir de la 3-fluoro-4-méthylaniline selon la méthode illustrée par les étapes 11.i., 11.j. et 11.k. de l'exemple 11. Solide jaune ; p.f. > 250° C.
RMN-¹H (DMSO) : 0,89 (t, 3H) ; 1,85 (q, 2H) ; 2,49 (s, 3H) ; 3,08 (d, 1H) ; 3,49 (d, 1H) ; 5,21 (s, 2H) ; 5,39 (d, 1H) ; 5,51 (d,1H) ; 6,05 (s, 1H) ; 7,39 (s, 1H) ; 7,87 (d, 1H) ; 8,05 (d, 1H) ; 8,61 (s, 1H).
RMN-C¹³(DMSO) : 8,40 ; 15,14 ; 36,45 ; 42,52 ; 50,60 ; 61,41 ; 73,28 ; 99,71 ; 112,00 ; 122,66 ; 125,38 ; 127,66 ; 129,59 ; 130,28 ; 144,49 ; 147,88 ; 152,88 ; 155,85 ; 159,18 ; 162,25 ; 172,02.
IR (KBr) : 1054 ; 1580 ; 1651 ; 1760.

### Exemple 24 : 5-éthyl-10-fluoro-4,5-dihydro-5-hydroxy-1H-oxepino [3',4':6,7] indolizino[1,2-b]quinoléine-3,15(4H,13H)-dione

Ce composé est obtenu à partir de la 4-fluoroaniline selon la méthode illustrée par les étapes 11.i., 11.j. et 11.k. de l'exemple 11. Solide blanc ; p.f. > 250° C.
RMN-¹H (DMSO) : 0,85 (t, 3H) ; 1,85 (q, 2H) ; 3,07 (d, 1H) ; 3,45 (d, 1H) ; 5,29 (s, 2H) ; 5,39 (d, 1H) ; 5,55 (d, 1H) ; 6,30 (s, 1H) ; 7,39 (s, 1H) ; 7,80 (q, 1H) ; 7,99 (q, 1H) ; 8,23 (q, 1H) ;8,68 (s, 1H).
RMN-C¹³ (DMSO) : 8,40 ; 36,46 ; 42,48 ; 50,66 ; 61,41 ; 73,31 ; 99,68 ; 111,83 ; 122,75 ; 128,93 ; 130,93 ; 131,22 ; 131,93 ; 144,46 ; 145,27 ; 152,60 ; 155,89 ; 159,21 ; 172,04.
IR (KBr) : 1209 ; 1589 ; 1659 ; 1739.

### Exemple 25 : 10-chloro-5-éthyl-4,5-dihydro-5-hydroxy-1H-oxepino [3',4':6,7]indolizino[1,2-b]quinoléine-3,15(4H,13H)-dione

Ce composé est obtenu à partir de la 4-chloroaniline selon la méthode illustrée par les étapes 11.i.,11 .j. et 11.k. de l'exemple 11. Solide jaune. p.f > 250° C.
RMN-¹H (DMSO) : 0,85 (t, 3H) ; 1,85 (q, 2H) ; 3,07 (d, 1H) ; 3,47 (d, 1H) ; 5,25 (s, 2H) ; 5,39 (d, 1H) ; 5,51 (d, 1H) ; 6,05 (s, 1H) ; 7,39 (s, 1H) ; 7,89 (d, 1H) ; 8,19 (d, 1H) ; 8,29 (s, 1H) ; 8,67 (s,1H).
RMN-C¹³ (DMSO) : 8,40 ; 36,46 ; 42,47 ; 50,70 ; 61,42 ; 73,31 ; 100,00 ; 122,96 ; 127,31 ; 127,42 ; 128,87 ; 131,11 ; 132,12 ; 144,34 ; 146,53 ; 153,38 ; 155,88 ; 159,20 ; 172,04.
IR (KBr) : 1069 ;1483 ; 1606 ; 1741.

### Exemple 26 : 10-chloro-5-éthyl-9-fluoro-4,5-dihydro-5-hydroxy-1H-oxepino [3',4':6,7]indolizino[1,2-b]quinoléine-3,15(4H,13H)-dione

Ce composé est obtenu à partir de la 4-chloro-3-fluoroaniline selon la méthode illustrée par les étapes 11.i., 11.j. et 11.k. de l'exemple 11. Solide jaune. p.f. > 250° C.
RMN-¹H (DMSO) : 0,85 (t, 3H) ; 1,85 (q, 2H) ; 3,07 (d, 1H) ; 3,45 (d, 1H) ; 5,25 (s, 2H) ; 5,39 (d, 1H) ; 5,51 (d, 1H) ; 6,05 (s, 1H) ; 7,40 (s, 1H) ; 8,20 (d, 1H) ; 8,40 (d, 1H) ; 8,68 (s, 1H).
RMN-C¹³ (DMSO) : 8,38 ; 36,47 ; 42,58 ; 50,71 ; 61,40 ; 73,26 ; 99,99 ; 113,59 ; 123,09 ; 124,28 ; 127,74 ; 130,64 ; 131,31 ; 144,13 ; 145,08 ; 153,57 ; 154,13 ; 155,84 ; 156,61 ; 159,14 ; 172,00.
IR (KBr) : 1488 ; 1583 ; 1655 ; 1743.

### Exemple 27 :5,12-diéthyl-4,5-dihydro-5,10-dihydroxy-11-morpholino méthyl-1H-oxepino[3',4':6,7]indolizino[1,2-b]quinoléine-3,15 (4H,13H)-dione

Ce composé est obtenu à partir de la morpholine selon la méthode illustrée dans l'exemple 15.a. Solide blanc, p.f. > 250° C.
RMN-¹H (DMSO) : 0,85 (t, 3H) ; 1,87 (q, 2H) ; 2,53 (s, 4H) ; 3,03 (d, 1H) ; 3,45 (d, 1H) ; 3,57 (s, 4H) ; 4,02 (s, 2H) ; 5,01 (s, 2H) ; 5,38 (d, 1H) ; 5,52 (d, 1H) ; 6,0 (se, 1H) ; 7,30 (s, 1H) ; 7,42 (d, 1H) ; 7,95 (d, 1H) ; 8,82 (s, 1H).
RMN-C¹³ (DMSO) : 8,45 ; 3,49 ; 42,58 ; 53,04 ; 61,44 ; 66,33 ; 73,33 ; 98,81 ; 113,78 ; 121,81 ; 122,74 ; 126,80 ; 129,05 ; 129,91 ; 143,72 ; 145,07 ; 149,24 ; 155,06 ; 156,92 ; 159,28 ; 172,08.
IR (KBr) : 1515 ; 1595 ; 1654 ; 1736.

### Exemple 28 : 5,12-diéthyl-9-fluoro-4,5-dihydro-5-hydroxy-10-méthoxy-1H-oxepino[3',4':6,7]indolizino[1,2-b]quinoléine-3,15(4H,13H)-dione

### 28.a. 5-fluoro-4-méthoxy-2-propionylaniline

(Ce produit est obtenu selon Sugasawa T; Toyoda T; Adachi M; Sasakura K, *J. Am. Chem*. *Soc.,* 100 (1978), p4842-4852). A une solution de 3-fluoro-4-méthoxy-aniline (20 g, 142 mmol) dans du dichlorométhane anhydre (200 ml), sous atmosphère d'argon et à 0° C, est ajouté goutte à goutte du trichlorure de bore (1M dans l'heptane, 156 ml, 156 mmol). La suspension rose ainsi obtenue est maintenue sous agitation 5 min puis sont ajoutés goutte à goutte du propionitrile (33 ml, 420 mmol) suivi de trichlorure d'aluminium (20,8 g, 156 mmol) par petites portions. Le milieu réactionel est chauffé au reflux pendant 3 h, refroidi à 0° C, hydrolysé en ajoutant avec précaution de l'acide chlorhydrique 2N (100 ml), puis porté au reflux 45 min. Après refroidissement à 0° C, on obtient un précipité qui est filtré, lavé au dichloromethane, puis repris dans de l'eau (300 ml). La phase aqueuse est basifiée jusqu'à pH alcalin, extraite au dichlorométhane puis à l'acétate d'éthyle. La phase organique est séchée (MgSO₄) puis évaporée pour donner un produit brut qui est purifié par chromatographie sur colonne (SiO₂, AcOEt/Hpt: 1/99 à 20/80). On obtient 15,3 g d'un solide jaune.
RMN-¹H (CDCl₃) : 1,20 (t, 3H) ; 2,92 (q, 2H) ; 3,83 (s, 3H) ; 6,2 (s, 2H) ; 6,40 (d, 2H) ; 7,32 (d, 2H).
IR (KBr) : 857 ; 1148 ; 1240 ; 1561 ; 1583 ; 1662.

### 28.b. 4-éthyl-7-fluoro-2-hydroxy-6-méthoxy-3-quinoléinecarboxylate d'éthyle

A une solution de 5-fluoro-4-méthoxy-2-propionylaniline (15,3 g, 77,5 mmol) et de triéthylamine (13,9 ml, 100 mmol) dans de l'acétonitrile anhydre (110 ml), sous argon et à 0° C, est ajoutée goutte à goutte une solution de chlorure d'éthylmalonyle (12,9 ml, 100 mmol) dans de l'acétonitrile anhydre (30 ml). On laisse le milieu réactionel revenir à température ambiante, on canule goutte à goutte et sous argon une solution d'éthylate de sodium (obtenue par 1,8 g, 78 mmol, de sodium dans 80 ml d'éthanol), puis on laisse sous agitation 12 h à température ambiante. On verse le mélange réactionnel dans de l'eau glacée (100 ml) et on agite deux heures, puis on filtre le précipité qui est lavé à l'eau, à l'éthanol et à l'éther. On obtient 19,4 g d'un solide blanc.
RMN-¹H (DMSO) : 1,25 (m, 6H) ; 2,78 (q, 2H) ; 3,92 (s, 3H) ; 4,30 (q, 2H) ; 7,15 (d, 2H) ; 7,40 (d, 2H) ; 11,93 (s, 1H).
IR (KBr) : 786 ; 1083 ; 1410 ; 1521 ; 1644 ; 1725.

### 28.c. 2-chloro-4-éthyl-7-fluoro-6-méthoxy-3-quinoléinecarboxylate d'éthyle

On porte au reflux pendant 6 h une suspension de 4-éthyl-7-fluoro-2-hydroxy-6-méthoxy-3-quinoléinecarboxylate d'éthyle (19,4 g, 0,066 mol) dans du chlorure de phosphoryle (243 ml). On distille le chlorure de phosphoryle. On transvase le mélange réactionnel dans de l'eau glacée. On reprend au dichlorométhane pour solubiliser. On lave la phase organique à l'eau, puis avec une solution saturée de chlorure de sodium. On sèche la phase organique sur sulfate de magnésium et on évapore le solvant. On suspend le résidu dans de l'éther et filtre du produit de départ non converti (4 g). On évapore le filtrat et purifie le résidu par chromatographie sur colonne (SiO₂, AcOEt/Hpt: 5/95 à 20/80). On obtient 10,9 g d'un solide blanc.
RMN-¹H (DMSO) : 1,30 (t, 3H) ; 1,39 (t, 3H) ; 3,08 (q, 2H) ; 4,09 (s, 3H) ; 4,49 (q, 2H) ; 7,64 (d, 2H) ; 7,86 (d, 2H).
IR (KBr) : 865 ; 1016 ; 1082 ; 1190 ; 1224 ; 1253 ; 1272 ; 1508 ; 1571 ; 1732.

### 28.d. 2-chloro-4-éthyl-7-fluoro-6-méthoxy-3-quinoléineméthanol

Une solution de 2-chloro-4-éthyl-7-fluoro-6-méthoxy-3-quinoléinecarboxylate d'éthyle (10,8 g, 35 mmol) dans du dichlorométhane anhydre (200 ml) est traitée goutte à goutte à température ambiante sous atmosphère inerte par de l'hydrure de diisobutylaluminium (1M dans du dichlorométhane, 65 ml, 65 mmol), puis chauffée à 40° C pendant 4 h. On refroidit à 0° C, on ajoute avec précaution une solution aqueuse de sel de Rochelle à 20 % (105 ml) et du dichlorométhane (200 ml) et on maintient sous agitation pendant 1 h. On décante, on lave trois fois à l'eau, on sèche la phase organique sur sulfate de magnésium et on évapore le solvant. Le résidu est purifié par chromatographie sur colonne (SiO₂, AcOEt/Hpt: 5/95 à 50/50). On obtient 6 g d'un solide blanc.
RMN-¹H (DMSO) : 1,28 (t, 3H) ; 3,25 (q, 2H) ; 4,04 (s, 3H) ; 4,77 (d, 2H) ; 5,27 (t, 1H) ; 7,55 (d, 2H) ; 7,73 (d, 2H).
IR (KBr) : 840 ; 864 ; 1023 ; 1232 ; 1267 ; 1317 ; 1444 ; 1511 ; 1569.

### 28.e. 5,12-diéthyl-9-fluoro-4,5-dihydro-5-hydroxy-10-méthoxy-1H-oxepino[3',4':6,7]indolizino[1,2-b]quinoléine-3,15(4H,13H)-dione

Le 2-chloro-4-éthyl-7-fluoro-6-méthoxy-3-quinoléineméthanol est couplé au composé (M) comme décrit dans l'étape 11.j. de l'exemple 11. Le produit de couplage résultant est cyclisé selon la procédure de l'étape 11.k. On obtient un solide jaune, p.f. > 275° C.
RMN-¹H (CF3COOD): 1,07 (m, 3H) ; 1,62 (m, 3H) ; 2,27 (m,2H) ; 3,44 (d, 1H) ; 3,54 (m, 2H) ; 3,91 (d, 1H) ; 4,25 (s, 3H) ; 5,60 (d, 1H) ; 5,74 (s, 2H) ; 5,98 (d, 1H) ; 7,85 (m, 1H) ; 8,16 (m, 1H) ; 8,31 (s, 1H).
RMN-C¹³ (CF3COOD) : 9,03 ; 14,20 ; 26,68 ; 38,77 ; 43,98 ; 53,79 ; 58,27 ; 64,73 ; 77,93 ; 106,85 ; 109,24 ; 110,15 ; 128,99 ; 129,20 ; 131,61 ; 137,32 ; 141,23 ; 144,13 ; 154,79 ; 158,32 ; 160,25 ; 160,81 ; 179,30.
IR (KBr) : 1013 ; 1068 ; 1265 ; 1466 ; 1514 ; 1601 ; 1655 ; 1748.

### Exemple 29 : 5-éthyl-4,5-dihydro-5-hydroxy-12-méthyl-1H-oxepino [3',4';6,7]indolizino[1,2-b]quinoléine-3,15(4H,13H)-dione

On applique à la 2-acétylaniline la procédure décrite par les exemples 28.b., 28.c. et 28.d. pour donner du 2-chloro-4-méthyl-3-quinoléineméthanol. Ce dernier est couplé au composé (M) comme décrit dans l'étape 11.j. de l'exemple 11. Le produit de couplage résultant est cyclisé selon la procédure de l'étape 11.k. On obtient un solide jaune, p.f. > 260° C.
RMN ¹H (DMSO) : 0,87 (t, 3H) ; 1,87 (q, 2H) ; 2,78 (s, 3H) ; 2,80 (d, 1H) ; 3,55 (d, 1H) ; 5,27 (s, 2H) ; 5,42 (d, 1H) ; 5,52 (d, 1H) ; 6,04 (s, 1H) ; 7,39 (s, 1H) ; 7,75 (t, 1H) ; 7,88 (t, 1H) ; 8,13 (d, 1H) ; 8,25 (d, 1H).
RMN-C¹³ (DMSO) : 8,23 ; 36,26 ; 42,36 ; 62,00 ; 73,11 ; 78,65 ; 79,13 ; 79,25 ; 99,52 ; 122,36 ; 124,30 ; 127,67 ; 129,54 ; 129,55 ; 129,56 ; 140,11 ; 145,06 ; 148,07 ; 152,00 ; 155,79 ; 159,09 ; 171,89.
IR (KBr) : 1649 ; 1751 ; 3404.

### Exemple 30 : 9-chloro-5-éthyl-4,5-dihydro-5-hydroxy-10-méthoxy-12-(4-méthyl piperazinométhyl)-1H-oxepino[3',4':6,7]indolizino [1,2-b] quinoléine-3,15(4H,13H)-dione

### 30.a. 5-chloro-2-chloroacétyl-4-méthoxyaniline

Ce produit est obtenu selon Sugasawa T. ; Toyoda T. ; Adachi M. ; Sasakura K. , *J*. *Am*. *Chem*. *Soc.,* 100 (1978), p. 4842-4852). A une solution de 3-chloro-4-méthoxy-aniline (23,6 g, 150 mmol) sous atmosphère inerte à 0° C sont ajoutés successivement, goutte à goutte, une solution molaire de trichlorure de bore dans l'hexane (164 ml, 164 mmol), du chloroacétonitrile (11,4 ml, 180 mmol), et une solution molaire de chlorure de diéthylaluminium dans l'hexane (164 ml, 164 mmol). Le milieu réactionel est chauffé au reflux pendant 1 h, refroidi à 0° C, hydrolysé en ajoutant avec précaution de l'acide chlorhydrique 2N (90 ml), puis porté au reflux 1 h. On refroidit et on ajoute une solution concentrée de soude jusqu'au pH 14. On extrait à l'acétate d'éthyle, on lave la phase organique à l'eau, puis à l'eau salée. On sèche sur sulfate de magnésium, on filtre et on évapore sous pression réduite. On reprend le résidu à l'isopentane, on décante, puis on reprend l'insoluble dans le minimum d'éther isopropylique, on ajoute de l'isopentane pour précipiter le produit. On filtre et on sèche sous vide. On obtient 17,26 g d'un solide brun.
RMN-¹H (CDCl₃) : 3,82 (s, 3H) ; 4,60 (s, 2H) ; 6,11 (s, 2H) ; 6,78 (s, 1H) ; 7,11 (s, 1H).

### 30.b. 7-chloro-4-chlorométhyl-2-hydroxy-6-méthoxy-3-quinoléine-carboxylate d'éthyle

A une solution de 5-chloro-2-chloroacétyl-4-méthoxyaniline (17 g, 73 mmol) et de triéthylamine (18,5 ml, 131 mmol) dans de l'acétonitrile anhydre (310 ml), sous argon et à 0° C, on ajoute goutte à goutte une solution de chlorure d'éthylmalonyle (17 ml, 131 mmol). On agite pendant 2 h à température ambiante, puis on ajoute goutte à goutte à 0° C une solution d'éthanolate de sodium dans l'éthanol (obtenue par 1,88 g, 80 mmol, de sodium dans 90 ml d'éthanol). On agite 12 heures à température ambiante. On ajoute 300 ml d'eau, on agite à nouveau pendant 20 minutes. On filtre le précipité ; on lave à l'eau, à l'éthanol, et à l'éther éthylique. On obtient après séchage sous vide 16,7 g d'un solide jaunâtre.
RMN-¹H (DMSO) : 1,31 (t, 3H) ; 3,95 (s, 3H) ; 4,36 (q, 2H) ; 4,95 (s, 2H) ; 7,46 (s, 1H) ; 7,49 (s,1H).

### 30.c. 2,7-dichloro-4-chlorométhyl-6-méthoxy-3-quinoléine-carboxylate d'éthyle

On porte au reflux pendant 6 h une suspension de 7-chloro-4-chlorométhyl-2-hydroxy-6-méthoxy-3-quinoléinecarboxylate d'éthyle (116,7 g, 50 mmol) dans du chlorure de phosphoryle (100 ml). On distille le chlorure de phosphoryle. On reprend le résidu à l'eau, et on agite 30 min. On filtre le précipité et on lave à l'eau jusqu'à neutralité. On reprend le précipité au dichlorométhane et avec une solution saturée de chlorure de sodium. On filtre sur lit de celite et on décante le filtrat. On lave à nouveau la phase organique par une solution saturée de chlorure de sodium. On sèche sur sulfate de magnésium, on filtre et on évapore sous pression réduite. On obtient 15,88 g d'une huile brune.
RMN-¹H (CDCl₃) : 1,47 (t, 3H) ; 4,08 (t, 3H) ; 4,55 (q, 2H) ; 4,87 (s, 2H) ; 7,35 (s, 1 H) ; 8,09 (s, 1H).

### 30.d. 2,7-dichloro-6-méthoxy-4-(4-méthylpipérazinométhyl)-3-quinoléine-carboxylate d'éthyle

Un mélange de 2,7-dichloro-4-chlorométhyl-6-méthoxy-3-quinoléine-carboxylate d'éthyle (6,9 g, 20 mmol) et de *N*-méthylpiperazine (9 ml, 80 mmol) est chauffé à 60° C pendant 30 min. On dilue la masse réactionnelle à l'eau et on extrait à l'acétate d'éthyle. On décante, on lave la phase organique à l'eau. On sèche sur sulfate de magnésium, on filtre et on évapore sous pression réduite. On reprend le résidu à l'eau, on agite 15 minutes, on filtre, on lave à l'eau et on sèche sous vide. On purifie le résidu par chromatographie sur colonne (SiO₂, MeOH/CH₂Cl₂: 5/95 à 8/92). On obtient 6,7 g de produit, un solide beige.
RMN-¹H (CDCl₃) : 1,45 (t, 3H) ; 2,28 (s, 3H) ; 2,35-2,70 (m, 8H) ; 3,86 (s, 2H) ; 4,04 (s, 3H) ; 4,48 (q, 2H) ; 7,77 (s, 1H) ; 8,05 (s, 1H).

### 30.e. 2,7-dichloro-6-méthoxy-4-(4-méthylpipérazinométhyl)-3-quinoléine-méthanol

On dissout du 2,7-dichloro-6-méthoxy-4-(4-méthylpipérazinométhyl)-3-quinoléine carboxylate d'éthyle (6 g, 14,5 mmol) dans du chlorure de méthylène (120 ml). On ajoute lentement une solution molaire d'hydrure de diisobutylaluminium dans le chlorure de méthylène (60 ml, 60 mmol). On agite une heure à température ambiante. On verse lentement la masse réactionnelle dans 300 ml d'une solution à 20 % de sel de Rochelle. On agite une heure ; on filtre sur celite ; on décante, on lave la phase organique avec une solution saturée de chlorure de sodium. On sèche sur sulfate de magnésium, on filtre et on évapore sous pression réduite. On reprend le solide avec de l'éther isopropylique, on filtre et on sèche sous vide. On obtient 4,3 g du produit recherché (80 %), sous forme d'un solide jaune.
RMN-¹H (CDCl₃) : 2,27 (s, 3H) ; 2,30-2,80 (m, 8H) ; 4,03 (s, 3H) ; 4,08 (s, 2H) ; 4,96 (s, 2H) ; 5,95 (s, 1H) ; 7,37 (s, 1H) ; 8,05 (s, 1H).

### 30.f. 9-chloro-5-éthyl-4,5-dihydro-5-hydroxy-10-méthoxy-12-(4-méthyl pipérazinométhyl)-1H-oxepino[3',4:6,7]indolizino[1,2-b]quinoléine-3,15(4H,13H)-dione

Le 2,7-dichloro-6-méthoxy-4-(4-méthylpipérazinométhyl)-3-quinoléine-méthanol est couplé au composé (M) comme décrit dans l'étape 11.j. de l'exemple 11. Le produit de couplage résultant est cyclisé selon la procédure de l'étape 11.k. On obtient un solide jaune, p.f. > 250° C.
RMN-¹H (DMSO) : 0,87 (t, 3H) ; 1,84 (q, 2H) ; 2,53 (s, 4H) ; 3,08 (d, 1H) ; 3,47 (d, 1H) ; 3,58 (s, 4H) ; 4,06 (s, 5H); 5,30 (s, 2H) ; 5,42 (q, 2H) ; 6,03 (s, 1H) ; 7,31 (s, 1H) ; 7,91 (s, 1H) ; 8,16 (s, 1H).
RMN-C¹³ (DMSO) : 8,42 ; 36,53 ; 50,65 ; 53,30 ; 56,67 ; 62,00 ; 66,50 ; 73,32 ; 99,31 ; 104,86 ; 122,32 ; 126,94 ; 127,70 ; 129,83 ; 130,44 ; 138,89 ; 144,22 ; 144,85 ; 151,05 ; 153,17 ; 155,92 ; 159,19 ; 172,06.
IR (KBr) : 862 ; 1063 ; 1116 ; 1248 ; 1595 ; 1655 ; 1744 ; 3449.

### Exemple 31 : 9-chloro-5-éthyl-4,5-dihydro-5-hydroxy-10-méthoxy-12-morpholinométhyl-1H-oxepino[3',4':6,7]indolizino[1,2-b]quinoléine-3,15(4H,13H)-dione

On applique à la 3-chloro-4-méthoxyaniline la procédure décrite par les exemples 30.a., 30.b. et 30.c. pour donner du 2,7-dichloro-4-chlorométhyl-6-méthoxy-3-quinoléine-carboxylate d'éthyle qui est traité selon la procédure de l'exemple 30.d., en utilisant la morpholine au lieu de la N-méthylpipérazine, puis réduit selon la méthode de l'exemple 30.e. en quinoléineméthanol correspondant. Ce dernier est couplé au composé (M) comme décrit dans l'étape 11.j. de l'exemple 11. Le produit de couplage résultant est cyclisé selon la procédure de l'étape 11.k. On obtient un solide beige, p.f. > 250° C.
RMN-¹H (DMSO) : 0,87 (t, 3H) ; 1,84 (q, 2H) ; 2,15 (s, 3H) ; 2,32 (s, 4H) ; 2,50 (s, 4H) ; 3,08 (d, 1H) ; 3,47 (d, 1H) ; 4,06 (s, 5H) ; 5,29 (s, 2H) ; 5,46 (q, 2H) ; 6,06 (s, 1H) ; 7,31 (s, 1H) ; 7,92 (s, 1H) ; 8,17 (s, 1H).
RMN-C¹³ (DMSO) : 8,42 ; 36,51 ; 42,57 ; 45,93 ; 50,66 ; 52,83 ; 55,05 ; 56,09 ; 56,72 ; 61,44 ; 73,29 ; 99,30 ; 104,89 ; 122,32 ; 126,89 ; 127,63 ; 129,85 ; 130,16 ; 138,78 ; 144,18 ; 144,81 ; 151,03 ; 153,10 ; 155,10 ; 159,17 ; 172,07.
IR (KBr) : 1055 ; 1252 ; 1596 ; 1655 ; 1747 ; 3449.

### Exemple 32 : 5-éthyl-4,5-dihydro-5-hydroxy-12-(4-méthyl pipérazinométhyl)-1H-oxepino[3',4':6,7] indolizino [1,2-b] quinoléine -3,15(4H,13H)-dione

On applique à l'aniline la procédure décrite par les exemples 30.a., 30.b. et 30.c. pour donner du 2-chloro-4-chlorométhyl-3-quinoléinecarboxylate d'éthyle qui est traité selon la procédure de l'exemple 30.d. avec la N-méthylpipérazine, puis réduit selon la méthode de l'exemple 30.e. en quinoléineméthanol correspondant. Ce dernier est couplé au composé (M) comme décrit dans l'étape 11.j. de l'exemple 11. Le produit de couplage résultant est cyclisé selon la procédure de l'étape 11.k. On obtient un solide jaune, p.f. > 260° C.
RMN-¹H (DMSO) : 0,86 (t, 3H) ; 1,87 (q, 2H) ; 2,14 (s, 3H) ; 2,32-2,60 (m, 8H) ; 3,05 (d, 1H) ; 3,48 (d, 1H) ; 4,09 (q, 2H) ; 5,42 (d, 1H) ; 5,52 (d, 1H) ; 6,03 (se, 1H) ; 7,40 (s, 1H) ; 7,72 (t, 1H) ; 7,85 (t, 1H) ; 8,16 (d, 1H) ; 8,45 (d, 1H).
IR (KBr) : 1652 ; 1735 ; 3424.

### Exemple 33 : 5-éthyl-4,5-dihydro-5-hydroxy-12-pipéridinométhyl-1H-oxepino [3',4':6,7]indolizino[1,2-b]quinoléine-3,15(4H,13H)-dione

On applique à l'aniline la procédure décrite par les exemples 30.a., 30.b. et 30.c. pour donner du 2-chloro-4-chlorométhyl-3-quinoléinecarboxylate d'éthyle qui est traité selon la procédure de l'exemple 30.d., en utilisant la pipéridine au lieu de la N-méthylpipérazine, puis réduit selon la méthode de l'exemple 30.e. en quinoléineméthanol correspondant. Ce dernier est couplé au composé (M) comme décrit dans l'étape 11.j. de l'exemple 11. Le produit de couplage résultant est cyclisé selon la procédure de l'étape 11.k. On obtient un solide jaune, p.f. > 260° C.
RMN-¹H (DMSO) : 0,86 (t, 3H) ; 1,40 (se, 2H) ; 1,48 (se, 4H) ; 1,87 (q, 2H) ; 2,50 (s, 4H) ; 3,05 (d, 1H) ; 3,48 (d, 1H) ; 4,04 (q, 2H) ; 5,33 (s, 2H) ; 5,42 (d, 1H) ; 5,51 (d, 1H) ; 6,07 (se, 1H) ; 7,75 (t, 1H) ; 7,85 (t, 1H) ; 8,15 (d, 1H) ; 8,45 (d, 1H).
RMN-C¹³ (DMSO) : 8,47 ; 23,50 ; 25,82 ; 36,50 ; 42,50 ; 50,68 ; 54,47 ; 58,00 ; 61,42 ; 73,35 ; 99,55 ; 122,61 ; 125,31 ; 127,58 ; 129,54 ; 129,55 ; 129,56 ; 129,57 ; 140,49 ; 144,95 ; 148,63 ; 152,41 ; 155,90 ; 159,23 ; 172,07.
IR (KBr) : 1659 ; 1727 ; 3408.

### Exemple 34 : 5-éthyl-4,5-dihydro-5-hydroxy-12-morpholinométhyl-1H-oxepino[3',4':6,7]indolizino[1,2-b]quinoléine-3,15(4H,13H)-dione

On applique à l'aniline la procédure décrite par les exemples 30.a., 30.b. et 30.c. pour donner du 2-chloro-4-chlorométhyl-3-quinoléinecarboxylate d'éthyle qui est traité selon la procédure de l'exemple 30.d, en utilisant la morpholine au lieu de la N-méthylpipérazine, puis réduit selon la méthode de l'exemple 30.e. en quinoléineméthanol correspondant. Ce dernier est couplé au composé (M) comme décrit dans l'étape 11.j. de l'exemple 11. Le produit de couplage résultant est cyclisé selon la procédure de l'étape 11.k. On obtient un solide jaune, p.f. > 260° C.
RMN-¹H (DMSO) : 0,86 (t, 3H) ; 1,87 (q, 2H) ; 3,05 (d, 1H) ; 3,30 (s, 4H) ; 3,49 (d, 1H) ; 3,55 (se, 4H) ; 4,10 (q, 2H) ; 5,35 (s, 2H) ; 5,40 (d, 1H) ; 5,54 (d, 1H) ; 6,04 (s, 1H) ; 7,72 (t, 1H) ; 7,85 (t, 1H) ; 8,16 (d, 1H) ; 8,47 (d, 1H).
RMN-C¹³ (DMSO) : 8,42 ; 36,51 ; 42,57 ; 50,68 ; 53,51 ; 56,06 ; 61,42 ; 66,41 ; 73,34 ; 99,56 ; 122,64 ; 125,25 ; 127,56 ; 129,81 ; 139,55 ; 144,92 ; 148,62 ; 152,39 ; 155,89 ; 159,21 ; 172,05.
IR (KBr) : 1657 ; 1729 ; 3347.

### Exemple 35 : 5-éthyl-10-fluoro-4,5-dihydro-5-hydroxy-12-(4-méthylpipérazinométhyl)-1H-oxepino[3',4':6,7]indolizino [1,2-b] quinoléine-3,15(4H,13H)-dione

On applique à la 4-fluoroaniline la procédure décrite par les exemples 30.a., 30.b. et 30.c. pour donner du 2-chloro-4-chlorométhyl-6-fluoro-3-quinoléinecarboxylate d'éthyle qui est traité selon la procédure de l'exemple 30.d. avec la N-méthylpipérazine, puis réduit selon la méthode de l'exemple 30.e. en quinoléineméthanol correspondant. Ce dernier est couplé au composé (M) comme décrit dans l'étape 11.j. de l'exemple 11. Le produit de couplage résultant est cyclisé selon la procédure de l'étape 11.k. On obtient un solide jaune, p.f. > 275° C.
RMN-¹H (DMSO) : 0,87 (t, 3H) ; 1,85 (q, 2H) ; 2,15 (s, 3H) ; 2,31 (m, 4H) ; 2,50 (m, 4H) ; 3,07 (d, 1H) ; 3,48 (d, 1H) ; 4,04 (m, 2H) ; 5,31 (s, 2H) ; 5,40 (d, 1H) ; 5,53 (d, 1H) ; 6,05 (s, 1H) ; 7,38 (s, 1H) ; 7,77 (m, 1H) ; 8,19 (m, 2H).
RMN-C¹³ (DMSO) : 8,43 ; 36,51 ; 42,54 ; 45,89 ; 50,67 ; 52,92 ; 54,93 ; 55,92 ; 73,32 ; 99,56 ; 122,69 ; 130,43 ; 132,40 ; 139,69 ; 144,70 ; 145,84 ; 152,19 ; 155,90 ; 159,17 ; 172,05.
IR (KBr) : 836 ; 1051 ; 1217 ; 1291 ; 1612 ; 1662 ; 1726.

### Exemple 36 : 5-ethyl-10-fluoro-4,5-dihydro-5-hydroxy-12-morpholinométhyl-1H-oxepino[3',4':6,7]indolizino[1,2-b]quinoléine-3,15(4H,13H)-dione

On applique à la 4-fluoroaniline la procédure décrite par les exemples 30.a., 30.b. et 30.c. pour donner du 2-chloro-4-chlorométhyl-6-fluoro-3-quinoléinecarboxylate d'éthyle qui est traité selon la procédure de l'exemple 30.d., en utilisant la morpholine au lieu de la N-méthylpipérazine, puis réduit selon la méthode de l'exemple 30.e. en quinoléineméthanol correspondant. Ce dernier est couplé au composé (M) comme décrit dans l'étape 11.j. de l'exemple 11. Le produit de couplage résultant est cyclisé selon la procédure de l'étape 11.k. On obtient un solide beige, p.f. > 250° C.
RMN-¹H (DMSO) : 0,87 (m, 3H) ; 1,85 (m, 2H) ; 2,51 (m,4H) ; 3,06 (d, 1H) ; 3,48 (d, 1H) ; 3,56 (m, 4H) ; 4,05 (m, 2H) ; 5,34 (s, 2H) ; 5,40 (d, 1H) ; 5,53 (d, 1H) ; 6,04 (s, 1H) ; 7,38 (s, 1H) ; 7,77 (m, 1H) ; 8,21 (m, 2H).
RMN-C¹³ (DMSO) : 8,40 ; 36,47 ; 42,52 ; 50,59 ; 53,40 ; 56,14 ; 61,44 ; 66,41 ; 73,29 ; 99,58 ; 109,05 ; 109,28 ; 120,11 ; 120,37 ; 122,68 ; 128,53 ; 130,53 ; 132,43 ; 139,13 ; 144,62 ; 145,79 ; 152,07 ; 155,94 ; 159,14 ; 161,59 ; 172,04.
IR (KBr) : 834 ; 860 ; 1061 ; 1118 ; 1215 ; 1286 ; 1516 ; 1609 ; 1658 ; 1734.

### Exemple 37 :5-éthyl-9-fluoro-4,5-dihydro-5-hydroxy-10-méthyl-12-(4-méthylpipérazinométhyl)-1H-oxepino[3',4':6,7]indolizino[1,2-b] quinoléine-3,15(4H,13H)-dione

On applique à la 3-fluoro-4-méthylaniline la procédure décrite par les exemples 30.a., 30.b. et 30.c. pour donner du 2-chloro-4-chlorométhyl-7-fluoro-6-méthyl-3-quinoléinecarboxylate d'éthyle qui est traité selon la procédure de l'exemple 30.d. avec la N-méthylpipérazine, puis réduit selon la méthode de l'exemple 30.e. en quinoléineméthanol correspondant. Ce dernier est couplé au composé (M) comme décrit dans l'étape 11.j. de l'exemple 11. Le produit de couplage résultant est cyclisé selon la procédure de l'étape 11.k. On obtient un solide jaune, p.f. > 260° C.
RMN-¹H (CDCl₃) : 1,00 (t, 3H) ; 2,00 (q, 2H) ; 2,35 (s, 3H) ; 2,50 (s, 3H) ; 2,61 (m, 8H) ; 3,33 (d, 1H) ; 3,39 (d, 1H) ; 3,97 (d, 1H) ; 4,07 (d, 1H) ; 5,17 (d, 1H) ; 5,38 (d, 1H) ; 5,52 (d, 1H) ; 5,63 (d, 1H) ; 7,13 (d, 1H) ; 7,28 (s, 1H) ; 7,99 (d, 1H).
IR (KBr) : 1652 ; 1747 ; 3430.

### Exemple 38: 5-éthyl-9-fluoro-4,5-dihydro-5-hydroxy-10-méthyl-12-morpholinométhyl-1H-oxepino[3',4':6,7]indolizino[1,2-b]quinoléine-3,15(4H,13H)-dione

On applique à la 3-fluoro-4-méthylaniline la procédure décrite par les exemples 30.a., 30.b. et 30.c. pour donner du 2-chloro-4-chlorométhyl-7-fluoro-6-méthyl-3-quinoléinecarboxylate d'éthyle qui est traité selon la procédure de l'exemple 30.d., en utilisant la morpholine au lieu de la N-méthylpipérazine, puis réduit selon la méthode de l'exemple 30.e. en quinoléineméthanol correspondant. Ce dernier est couplé au composé (M) comme décrit dans l'étape 11.j. de l'exemple 11. Le produit de couplage résultant est cyclisé selon la procédure de l'étape 11.k. On obtient un solide jaune, p.f. > 260° C.
RMN⁻¹H (DMSO + CDCl₃) : 1,00 (t, 3H) ; 2,02 (q, 2H) ; 2,57 (s, 3H) ; 2,60 (s, 4H) ; 3,23 (d, 1H) ; 3,45 (d, 1H) ; 3,75 (s, 4H) ; 4,11 (s, 2H) ; 5,44 (s, 2H) ; 5,47 (d, 1H) ; 5,65 (d, 1H) ; 7,62 (s, 1H) ; 7,73 (d, 1H) ; 8,24 (d, 1H).
RMN-C¹³ (CF₃CO₂D) : 8,35 ; 13,93 ; 16,01 ; 22,24 ; 25,29 ; 38,18 ; 43,42 ; 54,19 ; 56,04 ; 56,74 ; 64,16 ; 65,09 ; 77,48 ; 108,29 ; 108,57 ; 128,07 ; 128,70 ; 129,90 ; 135,64 ; 138,03 ; 139,86 ; 141,10 ; 141,56 ; 147,78 ; 158,30 ; 161,87 ; 178,72.
IR (KBr) : 117 ; 1609 ; 1654 ; 1750 ; 3437.

### Exemple 39 : 5-éthyl-9-fluoro-4,5-dihydro-5-hydroxy-10-méthyl-12-piperidinométhyl-1H-oxepino [3',4':6,7] indolizino [1,2-b] quinoléine-3,15(4H,13H)-dione

On applique à la 3-fluoro-4-méthylaniline la procédure décrite par les exemples 30.a., 30.b. et 30.c. pour donner du 2-chloro-4-chlorométhyl-7-fluoro-6-méthyl-3-quinoléinecarboxylate d'éthyle qui est traité selon la procédure de l'exemple 30.d., en utilisant la pipéridine au lieu de la N-méthylpipérazine, puis réduit selon la méthode de l'exemple 30.e. en quinoléineméthanol correspondant. Ce dernier est couplé au composé (M) comme décrit dans l'étape 11.j. de l'exemple 11. Le produit de couplage résultant est cyclisé selon la procédure de l'étape 11.k. On obtient un solide jaune, p.f. > 260° C.
RMN-¹H (CF₃CO₂D) : 1,09 (s, 3H) ; 1,70 (t, 1H) ; 2,03 (m, 5H) ; 2,25 (s, 2H) ; 2,70 (s, 3H) ; 3,54 (d, 3H) ; 3,88 (d, 1H) ; 4,01 (se, 2H) ; 5,30 (q, 2H) ; 5,65 (d, 1H) ; 5,96 (d, 1H) ; 6,10 (s, 2H) ; 8,16 (d, 1H) ; 8,35 (s, 1H) ; 8,61 (s, 1H).
RMN-C¹³ (CF₃CO₂D) : 8,47 ; 16,07 ; 20,93 ; 22,18 ; 24,76 ; 38,28 ; 43,53 ; 54,30 ; 56,12 ; 58,33 ; 64,24 ; 77,56 ; 108,37 ; 111,30 ; 128,20 ; 129,02 ; 129,98 ; 135,60 ; 138,29 ; 139,90 ; 141,60 ; 142,26 ; 147,57 ; 158,28 ; 161,90 ; 167,63 ; 170,31 ; 178,82.
IR (KBr) : 1605 ; 1657 ; 1728 ; 3399.

### Exemple 40 :8-éthyl-2,3,8,9-tétrahydro-8-hydroxy-16-(4-méthyl pipérazinométhyl)-10H,12H-[1,4] dioxino [2,3-g] oxepino [3',4':6,7] indolizino [1,2-b] quinoléine-10,13[15H]-dione

On applique à la 3,4-éthylènedioxyaniline la procédure décrite par les exemples 30.a., 30.b. et 30.c. pour donner du 2-chloro-4-chlorométhyl-6,7-éthylènedioxy-3-quinoléinecarboxylate d'éthyle qui est traité selon la procédure de l'exemple 30.d. avec la N-méthylpipérazine, puis réduit selon la méthode de l'exemple 30.e. en quinoléineméthanol correspondant. Ce dernier est couplé au composé (M) comme décrit dans l'étape 11.j. de l'exemple 11. Le produit de couplage résultant est cyclisé selon la procédure de l'étape 11.k. On obtient un solide jaune, p.f. > 260° C.
RMN-¹H (DMSO) : 0,92 (t, 3H) ; 1,89 (q, 2H) ; 2,16 (s, 3H) ; 2,50 (m, 8H) ; 3,12 (d, 1H) ; 3,50 (d, 1H) ; 3,95 (s, 2H) ; 4,47 (s, 4H) ; 5,19 (q, 2H) ; 5,43 (d, 1H) ; 5,56 (d, 1H) ; 7,35 (s, 1H) ; 7,54 (s, 1H) ; 7,76 (s, 1H).
RMN-C¹³ (DMSO) : 8,45 ; 24,80 ; 36,51 ; 42,48 ; 45,90 ; 50,45 ; 52,98 ; 54,91 ; 56,10 ; 61,44 ; 64,43 ; 73,30 ; 99,03 ; 109,46; 113,51 ; 121,95 ; 123,51 ; 127,76 ; 137,99; 145,00 ; 145,14 ; 145,27 ; 147,24 ; 150,53 ; 155,99 ; 159,18 ; 172,27 ; 177,00.
IR (KBr) : 1656 ; 1743 ; 3422.

### Exemple 41 : 9-chloro-5-éthyl-10-fluoro-4,5-dihydro-5-hydroxy-12-morpholinométhyl-1H-oxepino[3',4':6,7]indolizino[1,2-b]quinoléine-3,15(4H,13H)-dione

On applique à la 3-chloro-4-fluoroaniline la procédure décrite par les exemples 30.a., 30.b. et 30.c. pour donner du 2,7-dichloro-4-chlorométhyl-6-fluoro-3-quinoléinecarboxylate d'éthyle qui est traité selon la procédure de l'exemple 30.d., en utilisant la morpholine au lieu de la N-méthylpipérazine, puis réduit selon la méthode de l'exemple 30.e. en quinoléineméthanol correspondant. Ce dernier est couplé au composé (M) comme décrit dans l'étape 11.j. de l'exemple 11. Le produit de couplage résultant est cyclisé selon la procédure de l'étape 11.k. On obtient un solide beige, p.f. > 250° C.
RMN-¹H (CF₃COOD) : 1,09 (t, 3H) ; 2,30 (m, 2H) ; 3,50 (d, 1H) ; 3,90 (d, 1H) ; 3,98 (d, 4H) ; 4,36 (s, 4H) ; 5,38 (q, 2H) ; 5,64 (d, 1H) ; 5,96 (d, 1H) ; 6,23 (q, 2H) ; 8,57 (d, 1H) ; 8,60 (s, 1H) ; 8,85 (d, 1H).
RMN-C¹³ (CF₃COOD) : 8,10 ; 37,80 ; 43,11 ; 54,31 ; 55,78 ; 63,75 ; 65,11 ; 77,06 ; 128,28 ; 129,55 ; 130,33 ; 136,26 ; 137,11 ; 138,40 ; 139,67 ; 139,85 ; 148,58 ; 157,54 ; 159,74 ; 161,31 ; 178,00.
IR (KBr) : 848 ; 1042 ; 1230 ; 1609 ; 1658 ; 1750 ; 3310 ; 3387.

### Exemple 42 : résolution de la 5-éthyl-4,5-dihydro-5-hydroxy-1H-oxepino [3',4':6,7]-indolizine [1,2-b] quinoléine-3,15 (4H,13H)-dione

On porte à ébullition un mélange d'acide β-éthyl-β-hydroxy-(8-hydroxyméthylindolizino [1,2-*b*] quinoléine-9-(11*H*)-one-7-yl)-propionique (19,5 g, 51 mmol) et de L-(-)-α-méthylbenzylamine (12,12 g, 100 mmol) dans de l'éthanol absolu (1 l), on filtre à chaud et on laisse reposer 68 h. On filtre le précipité et on lave à l'éthanol et à l'éther pour donner 9,8 g d'un solide blanc. Une analyse par chromatographie liquide à haute pression sur phase stationnaire chirale ("HPLC chirale" sur colonne Chiral-AGP (Chromtech, Stockholm, Suède) 100 x 4 mm, éluant acétonitrile 2 % dans tampon phosphate 10 mM à pH 6,9, pics éluant à 4,5 et 7,5 min) révèle deux pics intégrant respectivement pour 24 % et 76 % de la surface totale des deux pics. On reprend le solide dans de l'éthanol à 93 % (350 ml) au reflux, puis on laisse reposer 48 h. On filtre le précipité puis on lave à l'éthanol et à l'éther pour obtenir 4,8 g d'un solide blanc donnant deux pics intégrant respectivement pour 9 % et 91 % de la surface totale des deux pics par HPLC chirale. On reprend le solide dans de l'éthanol à 50 % (48 ml) au reflux puis on laisse reposer 48 h. On filtre le précipité puis on lave à l'éthanol et à l'éther pour donner 2,7 g d'un solide blanc donnant deux pics intégrant respectivement pour 3 % et 97 % de la surface totale des deux pics par HPLC chirale. On reprend le solide dans de l'éthanol à 50 % (22 ml) au reflux puis on laisse reposer 48 h. On filtre le précipité puis on lave à l'éthanol et à l'éther pour obtenir 1,6 g d'un solide blanc donnant deux pics intégrant respectivement pour 1 % et 99 % de la surface totale des deux pics par HPLC chirale. On traite le sel résultant, diastéréoisomériquement enrichi, repris dans de l'eau distillée (20 ml), par de l'acide acétique (0,35 ml, 6,4 mmol) pendant 15 min. On filtre le précipité obtenu, on lave à l'eau, à l'acétone et à l'éther puis on sèche sous vide à 80° C pour obtenir 1,1 g d'un solide blanc. On reprend ce dernier dans de l'éthanol absolu (55 ml) additionné d'acide chlorhydrique concentré (11,5 N, 11 ml) pour obtenir une solution jaune qui est maintenue sous agitation à température ambiante 68 h. On filtre le précipité ainsi obtenu et on lave à l'eau, à l'éthanol et à l'éther, puis on sèche sous vide à 80° C pour obtenir 770 mg de 5-éthyl-4,5-dihydro-5-hydroxy-1*H*-oxepino [3',4':6,7]-indolizine [1,2-*b*] quinoléine-3,15 (4*H*,13*H*)-dione énantiomériquement enrichie. Une analyse par HPLC chirale (colonne Chiral-AGP, éluée par un gradient de 2 à 5 % d'acétonitrile dans tampon phosphate 10 mM à pH 6,9, pics éluant à 15 et 20 min) révèle un excès enantiomérique de 98 %. On reprend la procédure décrite ci-dessus en remplaçant la L-(-)-α-méthylbenzylamine par la D-(+)-α-méthylbenzylamine. On obtient ainsi l'autre énantiomère du 5-éthyl-4,5-dihydro-5-hydroxy-1*H*-oxepino [3',4':6,7]-indolizine [1,2-*b*] quinoléine-3,15 (4*H*,13*H*)-dione.

En utilisant les procédés indiqués ci-dessus, on peut également préparer les produits suivants, qui font également partie de l'invention et qui constituent des produits préférés :
10-benzyloxy-5,12-diéthyl-4,5-dihydro-5-hydroxy-1*H*-oxepino [3',4':6,7] indolizino [1,2-*b*] quinoléine-3,15 (4*H*, 13*H*)-dione ;
5,12-diéthyl-4,5-dihydro-5,10-dihydroxy-*1H*-oxepino [3', 4':6,7] indolizino [1,2-*b*] quinoléine 3,15 (4*H*, 13*H*)-dione ;
5,12-diéthyl-4,5-dihydro-5,10-dihydroxy-11-diméthylaminométhyl-1*H*-oxepino [3',4':6,7] indolizino [1,2-*b*] quinoléine-3,15 (4*H*, 13*H*)-dione ;
5-éthyl-4,5-dihydro-5-hydroxy-9,10-diméthoxy-1*H*-oxepino [3',4':6,7] indolizino [1,2-*b*] quinoléine-3,15(4*H*, 13*H*)-dione ;
10-bromo-5-éthyl-4,5-dihydro-5-hydroxy-1*H*-oxepino [3',4':6,7] indolizino [1,2-*b*] quinoléine-3,15(4*H*, 13*H*)-dione ;
11-bromo-5-éthyl-4,5-dihydro-5-hydroxy-10-méthoxy-1*H*-oxepino [3',4':6,7] indolizino [1,2-*b*] quinoléine-3,15 (4*H*, 13*H*)-dione ;
5-éthyl-12-diéthylaminométhyl-4,5-dihydro-5-hydroxy-1*H*-oxepino [3',4':6,7] indolizino [1,2-*b*] quinoléine-3,15 (4*H*, 13*H*)-dione ;
5-éthyl-4,5-dihydro-5-hydroxy-1*H*,3*H*-cyclopenta [*g*] oxepino [3',4':6,7] indolizino [1,2-*b*] quinoléine-3,16 [14*H*]-dione ;
7-éthyl-7,8-dihydro-7-hydroxy-16-(4-méthylpiperazino méthyl)-9*H*, 11*H*-[1,3] dioxolo [4,5-*g*] oxepino [3',4':6,7] indolizino [1,2-*b*] quinoléine-9,12 [14*H*]-dione ;
5-éthyl-4,5-dihydro-5-hydroxy-12-(1-imidazylméthyl)-1*H*-oxepino [3',4':6,7] indolizino [1,2-*b*] quinoléine-3,15 [4*H*, 13*H*]-dione ;
8-éthyl-8,9-dihydro-8-hydroxy-10*H*, 12*H*-oxepino [3',4':6,7] indolizino [1,2-*b*] pyrido [2,3-*g*] quinoléine-10,13 [15*H*]-dione ;
5-éthyl-4,5,9,10,11,12-hexahydro-5-hydroxy-1*H*, 3*H*-benzo [*g*] oxepino [3',4':6,7] indolizino [1,2-*b*] quinoléine-3,17 [15*H*]-dione ;
8-éthyl-8,9-dihydro-8-hydroxy-14*H*, 10*H*, 12*H* [1,3] dioxino [4,5-*g*] oxepino [3',4':6,7] indolizino [1,2-*b*] quinoléine-10,13 [15*H*]*-dione;*
7-éthyl-7,8-dihydro-7-hydroxy-1*H*, 9*H*, 11*H*-oxepino [3',4':6,7] indolizino [1,2-*b*] pyrrolo [2,3-*g*] quinoléine-9,12 [14*H*]-dione ;
7-éthyl-7,8-dihydro-7-hydroxy-1*H*, 9*H*, 11*H*-imidazo [4,5-*g*] oxepino [3',4':6,7] indolizino [1,2-*b*] quinoléine-9,12 [14*H*]-dione ;
7-éthyl-7,8-dihydro-7-hydroxy-1*H,* 9*H*, 11*H*-oxepino [3',4':6,7] indolizino [1,2-*b*] 1,2,3-triazolo [4,5-*g*] quinoléine-9,12 [14*H*]-dione ;
7-éthyl-7,8-dihydro-7-hydroxy-9*H*, 11*H*-oxepino [3',4':6,7] indolizino [1,2-*b*] thiazolo [4,5-*g*] quinoléine-9,12 [14*H*]-dione ; et
7-éthyl-7,8-dihydro-7-hydroxy-9*H*, 11*H*-oxazolo [4,5-*g*] oxepino [3',4':6,7] indolizino [1,2-*b*] quinoléine-9,12 [14*H*]-dione.

## Revendications

1. Procédé de préparation des composés de formule O 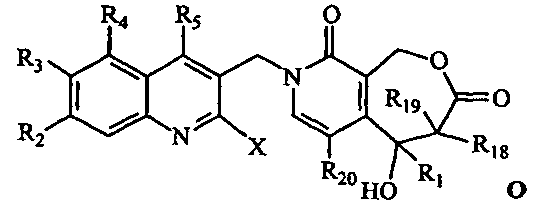 sous forme racémique, d'énantiomère ou toutes combinaisons de ces formes, dans laquelle
R₁ représente un alkyle inférieur, un alkényle inférieur, un alkynyl inférieur, un haloalkyle inférieur, un alkoxy inférieur alkyle inférieur ou un alkylthio inférieur alkyle inférieur ;
R₂, R₃ et R₄ représentent, indépendamment, H, halo, halo alkyle inférieur, alkyle inférieur, alkényle inférieur, cyano, cyano alkyle inférieur, nitro, nitro alkyle inférieur, amido, amido alkyle inférieur, hydrazino, hydrazino alkyle inférieur, azido, azido alkyle inférieur, -(CH₂)ₘNR₆R₇, -(CH₂)ₘOR₆, -(CH₂)ₘSR₆, -(CH₂)ₘCO₂R₆, -(CH₂)ₘNR₆C(O)R₈, -(CH₂)ₘC(O)R₈, -(CH₂)ₘOC(O)R₈, -O(CH₂)ₘNR₆R₇, -OC(O)NR₆R₇, -OC(O)(CH₂)ₘCO₂R₆, ou -(CH₂)ₙ[N=X], -OC(O)[N=X], -(CH₂)ₘOC(O)[N=X], aryle ou aryle alkyle inférieur substitué ou non substitué, dans lequel le substituant est un alkyle inférieur, halo, nitro, amino, alkylamino inférieur, haloalkyle inférieur, hydroxy alkyle inférieur, alkoxy inférieur, ou alkoxy inférieur alkyle inférieur, ou R₂ et R₃ forment ensemble une chaîne à 3 ou 4 chaînons, dans laquelle les éléments de la chaîne sont sélectionnés parmi le groupe constitué de CH, CH₂, O, S, N ou NR₉ ;
R₅ représente H, halo, halo alkyle inférieur, alkyle inférieur, alkoxy inférieur, alkoxy inférieur alkyle inférieur, alkylthio inférieur alkyle inférieur, cycloalkyle, cycloalkyle alkyle inférieur, cyano, cyano alkyle, alkyle inférieur sulphonyl alkyle inférieur, hydroxy alkyle inférieur, nitro, -(CH₂)ₘC(O)R₈, -(CH₂)ₘNR₆C(O)R₈, -(CH₂)ₘNR₆R₇, -(CH₂)ₘN(CH₃)(CH₂)ₙNR₆R₇, -(CH₂)ₘOC(O)R₈, -(CH₂)ₘOC(O)NR₆R₇, -(CH₂)ₘS(O)_{q}R₁₁, -(CH₂)ₘP(O)R₁₂R₁₃, -(CH₂)₂P(S)R₁₂R₁₃, ou un radical -(CH₂)ₙ[N=X], -OC(O)[N=X], -(CH₂)ₘOC(O)[N=X], aryle ou aryle alkyle inférieur substitué ou non substitué, dans lequel le substituant est un alkyle inférieur, halo, nitro, amino, alkyle inférieur amino, halo alkyle inférieur, hydroxy alkyle inférieur, alkoxy inférieur ou alkoxy inférieur alkyle inférieur ;
R₆ et R₇ représentent, indépendamment, H, un alkyle inférieur, hydroxy alkyle inférieur, alkyle inférieur amino alkyle inférieur, amino alkyle inférieur, cycloalkyle, cycloalkyle alkyle inférieur, alkényle inférieur, alkoxy inférieur alkyle inférieur, halo alkyle inférieur, le radical substitué ou non substitué aryle ou aryle alkyle inférieur, dans lequel le substituant est un alkyle inférieur, halo, nitro, amino, alkyle inférieur amino, halo alkyle inférieur, hydroxy alkyle inférieur, alkoxy inférieur, ou alkoxy inférieur alkyle inférieur ;
R₈ représente H, un alkyle inférieur, hydroxy alkyle inférieur, amino, alkyle inférieur amino, alkyle inférieur amino alkyle inférieur, amino alkyle inférieur, cycloalkyle, cycloalkyle alkyle inférieur, alkényle inférieur, alkoxy inférieur, alkoxy inférieur alkyle inférieur, halo alkyle inférieur, ou le radical substitué ou non substitué aryle ou aryle alkyle inférieur, dans lequel le substituant est un alkyle inférieur, halo, nitro, amino, alkyle inférieur amino, halo alkyle inférieur, hydroxy alkyle inférieur, alkoxy inférieur, ou alkoxy inférieur alkyle inférieur ;
R₉ représente H, un alkyle inférieur, halo alkyle inférieur, aryle, ou aryle substitué par un ou plusieurs groupes choisis parmi le radical alkyle inférieur, halo, nitro, amino, alkyle inférieur amino, halo alkyle inférieur, hydroxy alkyle inférieur, alkoxy inférieur, ou alkoxy inférieur alkyle inférieur ;
R₁₀ représente H, un alkyle inférieur, halo alkyle inférieur, alkoxy inférieur, aryle, ou aryle substitué par un ou plusieurs groupes choisis parmi le radical alkyle inférieur, halo alkyle inférieur, hydroxy alkyle inférieur, ou alkoxy inférieur alkyle inférieur ;
R₁₁ représente un alkyle inférieur, aryle, -(CH₂)ₘOR₁₄, -(CH₂)ₘSR₁₄, -(CH₂)₂NR₁₄R₁₅ ou -(CH₂)ₘ[N=X] ;
R₁₂ et R₁₃ représentent, indépendamment, un alkyle inférieur, aryle, alkoxy inférieur, aryloxy ou amino ;
R₁₄ et R₁₅ représentent, indépendamment, H, un alkyle inférieur ou aryle ;
R₁₈ et R₁₉ représentent, indépendamment, H, halo, alkyl inférieur, alkoxy inférieur ou hydroxy ;
R₂₀ représente H ou halo ;
m est un nombre entier compris entre 0 et 6 ;
n est 1 ou 2 ; et
q représente un nombre entier de 0 à 2 ;
[N=X] représente un groupe hétérocyclique de 4 à 7 chaînons, X représentant la chaîne nécessaire pour compléter ledit groupe hétérocyclique et sélectionnée dans le groupe constitué de O, S, CH₂, CH, N, NR₉ et COR₁₀ ; et
X représente un atome d'halogène, ou un sel pharmaceutiquement acceptable de ce dernier, étant entendu que le terme inférieur en référence aux groupes alkyle, alkylthio et alkoxy signifie un groupe comportant de 1 à 6 atomes de carbone et en référence aux groupes alkényle ou alkynyle, un groupe comportant de 2 à 6 atomes de carbone,
**caractérisé en ce que** l'on couple un composé de formule générale M 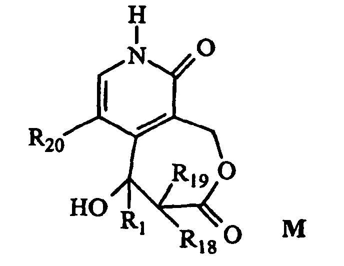 dans laquelle R₁, R₁₈, R₁₉ et R₂₀ ont la signification indiquée ci-dessus, avec un 2-halo-3-quinoléine-méthanol de formule générale N 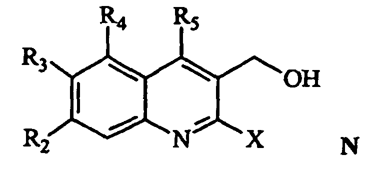 dans laquelle R₂, R₃, R₄, R₅ et X ont la signification indiquée ci-dessus, pour donner le composé de formule O.

2. Procédé de préparation selon la revendication 1, **caractérisé en ce que**
R₁ représente un alkyle inférieur, alkényle inférieur, halo alkyle inférieur ou alkoxy inférieur alkyle inférieur ;
R₂, R₃ et R₄ représentent, indépendamment, H, un halo, halo alkyle inférieur, alkyle inférieur, nitro, amido, amido alkyle inférieur, hydrazino, hydrazino alkyle inférieur, azido, azido alkyle inférieur, -(CH₂)ₘNR₆R₇, -(CH₂)ₘ,OR₆, -(CH₂)ₘSR₆, -(CH₂)ₘC(O)R₈, -OC(O)NR₆R₇, -(CH₂)ₙ[N=X] ou -(CH₂)ₘOC(O)[N=X] substitué ou non substitué, ou -OC(O)[N=X], ou R₂ et R₃, ensemble, forment une chaîne à 3 ou 4 chaînons, dans laquelle lesdits éléments de la chaîne sont choisis dans le groupe constitué de CH, CH₂, O, S, N ou NR₉;
R₅ représente H, halo, halo alkyle inférieur, alkyle inférieur, alkoxy inférieur, alkoxy inférieur alkyle inférieur, alkylthio inférieur alkyle inférieur, hydroxy alkyle inférieur, nitro, -(CH₂)ₘC(O)R₈, -(CH₂)ₘNR₆C(O)R₈, -(CH₂)ₘNR₆R₇, -(CH₂)ₘN(CH₃)(CH₂)ₙNR₆R₇, -(CH₂)ₘOC(O)R₈, -(CH₂)ₘOC(O)NR₆R₇, -(CH₂)ₙ[N=X] ou -OC(O)[N=X] substitué ou non substitué, ou -(CH₂)ₘOC(O)[N=X] ;
R₆ et R₇ représentent, indépendamment, H, un alkyle inférieur, hydroxy alkyle inférieur, alkyle inférieur amino alkyle inférieur, amino alkyle inférieur, cycloalkyle, cycloalkyle alkyle inférieur, alkoxy inférieur alkyle inférieur, aryle, aryle alkyle inférieur ou halo alkyle inférieur ;
R₈ représente H, un alkyle inférieur, hydroxy alkyle inférieur, alkyle inférieur amino, alkyle inférieur amino alkyle inférieur, amino alkyle inférieur, cycloalkyle, cycloalkyle alkyle inférieur, alkényle inférieur, alkoxy inférieur, alxoxy inférieur alkyle inférieur, halo alkyle inférieur, aryle ou aryle alkyle inférieur ;
R₉ représente H, un alkyle inférieur, ou un halo alkyle inférieur ;
R₁₀ représente H, un alkyle inférieur, halo alkyle inférieur, ou alkoxy inférieur ;
R₁₈, R₁₉ et R₂₀ représentent H.

3. Procédé de préparation selon l'une des revendications précédentes, **caractérisé en ce que** R₁ représente le groupe éthyle.

4. Procédé de préparation selon l'une des revendications précédentes, **caractérisé en ce que** R₂ et R₃ représentent, indépendamment, H, un alkyle inférieur, halo, halo alkyle inférieur ou -(CH₂)ₘOR₆, ou R₂ et R₃, ensemble, forment un méthylènedioxy ou un éthylènedioxy ; et R₄ et R₅ représentent, indépendamment, H, un alkyle inférieur, -(CH₂)ₘNR₆R₇ ou -(CH₂)ₙ[N=X] non substitué ou substitué par un alkyle inférieur.

5. Procédé de préparation selon l'une des revendications précédentes, **caractérisé en ce que** que R₄ représente H ou -(CH₂)ₘNR₆R₇ dans lequel R₆ et R₇ représentent, indépendamment, H ou un alkyle inférieur, et R₅ représente H, un alkyle inférieur ou -(CH₂)ₙ[N=X] substitué ou non substitué et [N=X] représente le goupe pipérazinyle ou morpholinyle et ledit substituant est un alkyle inférieur.

6. Procédé de préparation selon l'une des revendications précédentes, **caractérisé en ce que** R₂ représente H ou un halo ; et R₃ représente H, un alkyle inférieur, halo, ou OR₆ dans lequel R₆ représente H, un alkyle inférieur ou un aryle alkyle inférieur.

7. Procédé de préparation selon l'une des revendications précédentes, **caractérisé en ce que** R₂ représente H, chloro ou fluoro ; et R₃ représente H, fluoro, chloro, méthyle ou méthoxy.

8. Composés de formule O 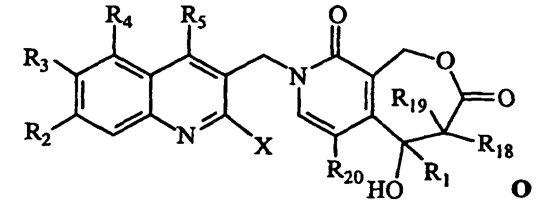 dans laquelle R₁, R₂, R₃, R₄, R₅, R₁₈, R₁₉, R₂₀ et X ont la signification indiquée à la revendication 1.

9. Utilisation des composés de formule O tels que définis à la revendication 8, pour la préparation des composés de formule I 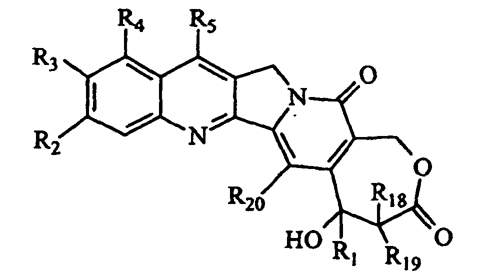 dans laquelle R₁, R₂, R₃, R₄, R₅, R₁₈, R₁₉ et R₂₀ ont la signification indiquée à la revendication 1, ou un sel thérapeutiquement acceptable de ce dernier,
**caractérisé en ce que** l'on cyclise le composé de formule générale O tel que défini à la revendication 8, pour obtenir le composé de formule générale I telle que définie ci-dessus.

10. Utilisation selon la revendication 9, pour la préparation d'un composé de formule (I), **caractérisé en ce que** ledit composé répond à la formule suivante : 5-éthyl-9,10-difluoro-4,5-dihydro-5-hydroxy-1*H*-oxepino[3',4':6,7] indolizino [1,2-*b*] quinoléine -3,15(4*H*,13*H*)-dione.

## Patentansprüche

1. Verfahren zur Herstellung der Verbindungen der Formel O 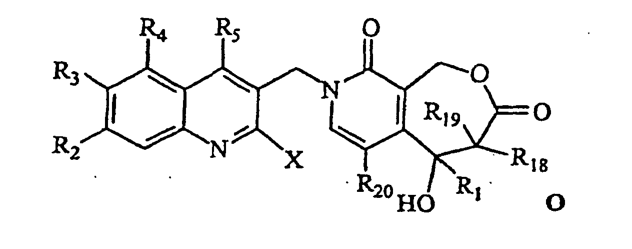 in Form von Racemat, von Enantiomer oder in allen Kombinationen dieser Formen, in welcher
R₁ Niederalkyl, Niederalkenyl, Niederalkinyl, Halogenniederalkyl, Niederalkoxyniederalkyl oder Niederalkylthioniederalkyl darstellt;
R₂, R₃ und R₄ unabhängig voneinander H, Halogen, Halogenniederalkyl, Niederalkyl, Niederalkenyl, Cyano, Cyanoniederalkyl, Nitro, Nitroniederalkyl, Amido, Amidoniederalkyl, Hydrazino, Hydrazinoniederalkyl, Azido, Azidoniederalkyl, -(CH₂)ₘNR₆R₇, -(CH₂)ₘOR₆, -(CH₂)ₘSR₆, -(CH₂)ₘCO₂R₆, -(CH₂)ₘNR₆C(O)R₈, -(CH₂)ₘC(O)R₈, -(CH₂)ₘOC(O)R₈, -O(CH₂)ₘNR₆R₇, -OC(O)NR₆R₇, -OC(O)(CH₂)ₘCO₂R₆ oder -(CH₂)ₙ[N=X], -OC(O) [N=X], -(CH₂)ₘOC(O) [N=X], Aryl oder substituiertes oder nicht substituiertes Arylniederalkyl darstellen, in dem der Substituent Niederalkyl, Halogen, Nitro, Amino, Niederalkylamino, Halogenniederalkyl, Hydroxyniederalkyl, Niederalkoxy oder Niederalkoxyniederalkyl ist, oder
R₂ und R₃ zusammen eine Kette mit 3 oder 4 Gliedern bilden, in der die Elemente der Kette aus der Gruppe ausgewählt sind, die aus CH, CH₂, O, S, N oder NR₉ besteht;
R₅ H, Halogen, Halogenniederalkyl, Niederalkyl, Niederalkoxy, Niederalkoxyniederalkyl, Niederalkylthioniederalkyl, Cycloalkyl, Cycloalkylniederalkyl, Cyano, Cyanoalkyl, Niederalkylsulphonylniederalkyl, Hydroxyniederalkyl, Nitro, -(CH₂)ₘC(O)R₈, -(CH₂)ₘNR₆C(O)R₈, -(CH₂)ₘNR₆R₇, -(CH₂)ₘN(CH₃) (CH₂)ₙNR₆R₇, -(CH₂)ₘOC(O)R₈, -(CH₂)ₘOC(O)NR₆R₇, -(CH₂)ₘS(O)_{q}R₁₁, -(CH₂)ₘP(O)R₁₂R₁₃, -(CH₂)₂P(S)R₁₂R₁₃ oder einen der Reste -(CH₂)ₘ [N=X], -OC(O) [N=X], -(CH₂)ₘOC(O) [N=X], Aryl oder substituiertes oder nicht substituiertes Arylniederalkyl darstellt, in dem der Substituent Niederalkyl, Halogen, Nitro, Amino, Niederalkylamino, Halogenniederalkyl, Hydroxyniederalkyl, Niederalkoxy oder Niederalkoxyniederalkyl ist;
R₆ und R₇ unabhängig voneinander H, Niederalkyl, Hydroxyniederalkyl, Niederalkylaminoniederalkyl, Aminoniederalkyl, Cycloalkyl, Cycloalkylniederalkyl, Niederalkenyl, Niederalkoxyniederalkyl, Halogenniederalkyl, einen substituierten oder nicht substituierten Aryl- oder Arylniederalkylrest darstellen, in dem der Substituent Niederalkyl, Halogen, Nitro, Amino, Niederalkylamino, Halogenniederalkyl, Hydroxyniederalkyl, Niederalkoxy oder Niederalkoxyniederalkyl ist;
R₈ H, Niederalkyl, Hydroxyniederalkyl, Amino, Niederalkylamino, Niederalkylaminoniederalkyl, Aminoniederalkyl, Cycloalkyl, Cycloalkylniederalkyl, Niederalkenyl, Niederalkoxy, Niederalkoxyniederalkyl, Halogenniederalkyl oder einen substituierten oder nicht substituierten Aryl- oder Arylniederalkylrest darstellt, in dem der Substituent Niederalkyl, Halogen, Nitro, Amino, Niederalkylamino, Halogenniederalkyl, Hydroxyniederalkyl, Niederalkoxy oder Niederalkoxyniederalkyl ist;
R₉ H, Niederalkyl, Halogenniederalkyl, Aryl, oder Aryl darstellt, das durch eine oder mehrere Gruppen substituiert ist, die aus den Resten Niederalkyl, Halogen, Nitro, Amino, Niederalkylamino, Halogenniederalkyl, Hydroxyniederalkyl, Niederalkoxy oder Niederalkoxyniederalkyl ausgewählt sind; R₁₀ H, Niederalkyl, Halogenniederalkyl, Niederalkoxy, Aryl oder Aryl darstellt, das durch eine oder mehrere Gruppen substituiert ist, die aus den Resten Niederalkyl, Halogenniederalkyl, Hydroxyniederalkyl oder Niederalkoxyniederalkyl ausgewählt sind;
R₁₁ Niederalkyl, Aryl, -(CH₂)ₘOR₁₄, -(CH₂)ₘSR₁₄, -(CH₂)₂NR₁₄R₁₅ oder -(CH₂)ₘ [N=X] darstellt;
R₁₂ und R₁₃ unabhängig voneinander Niederalkyl, Aryl, Niederalkoxy, Aryloxy oder Amino darstellen;
R₁₄ und R₁₅ unabhängig voneinander H, Niederalkyl oder Aryl darstellen;
R₁₈ und R₁₉ unabhängig voneinander H, Halogen, Niederalkyl, Niederalkoxy oder Hydroxy darstellen.;
R₂₀ H oder Halogen darstellt;
m eine ganze Zahl zwischen 0 und 6 ist;
n 1 oder 2 ist; und
q eine ganze Zahl von 0 bis 2 darstellt;
[N=X] eine heterocyclische Gruppe mit 4 bis 7 Gliedern darstellt, wobei X die zur Vervollständigung der heteröcyclischen Gruppe erforderliche Kette ist und aus der Gruppe ausgewählt ist, die aus O, S, CH₂, CH, N, NR₉ und COR₁₀ besteht; und
X ein Halogenatom darstellt, oder ein pharmazeutisch annehmbares Salz davon,
wobei gilt, dass der Begriff "Nieder" in Bezug auf die Gruppen Alkyl, Alkylthio und Alkoxy eine Gruppe mit 1 bis 6 Kohlenstoffatomen und in Bezug auf die Gruppen Alkenyl oder Alkinyl eine Gruppe mit 2 bis 6 Kohlenstoffatomen bedeutet,
**dadurch gekennzeichnet, dass** man die Verbindung der allgemeinen Formel M 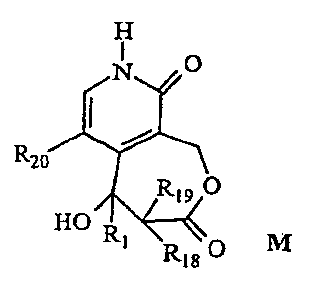 in der R₁, R₁₈, R₁₉ und R₂₀ die oben angegebene Bedeutung haben, mit einem 2-Halogen-3-chinolinmethanol der allgemeinen Formel N 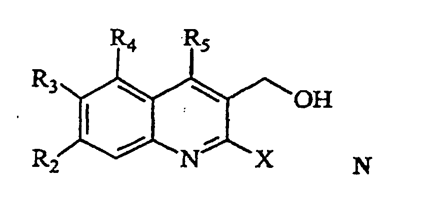 in der R₂, R₃, R₄, R₅ und X die oben angegebene Bedeutung haben, koppelt, um die Verbindung der Formel O zu erhalten.

2. Verfahren nach Anspruch 1, **dadurch gekennzeichnet, dass** R₁ Niederalkyl, Niederalkenyl, Halogenniederalkyl oder Niederalkoxyniederalkyl darstellt;
R₂, R₃ und R₄ unabhängig voneinander H, Halogen, Halogenniederalkyl, Niederalkyl, Nitro, Amido, Amidoniederalkyl, Hydrazino, Hydrazinoniederalkyl, Azido, Azidoniederalkyl, -(CH₂)ₘNR₆R₇, -(CH₂)ₘOR₆, -(CH₂)ₘSR₆, -(CH₂)ₘC(O)R₈, -OC(O)NR₆R₇, -(CH₂)ₙ [N=X] oder substituiertes oder nicht substituiertes -(CH₂)ₘOC(O) [N=X] oder -OC(O) [N=X] darstellen oder R₂ und R₃ zusammen eine Kette mit 3 oder 4 Gliedern bilden, in der die Elemente der Kette aus der Gruppe ausgewählt sind, die aus CH, CH₂, O, S, N oder NR₉ besteht; R₅ H, Halogen, Halogenniederalkyl, Niederalkyl, Niederalkoxy, Niederalkoxyniederalkyl, Niederalkylthioniederalkyl, Hydroxyniederalkyl, Nitro, -(CH₂)ₘC(O)R₈, -(CH₂)ₘNR₆C(O)R₈, -(CH₂)NR₆R₇, -(CH₂)ₘN(CH₃) (CH₂)ₙNR₆R₇, -(CH₂)ₘOC(O)R₈, -(CH₂)mOC(O)NR₆R₇, -(CH₂)ₙ [N=X] oder substituiertes oder nicht substituiertes -OC(O) [N=X] oder -(CH₂)ₘOC(O) [N=X] darstellt;
R₆ und R₇ unabhängig voneinander H, Niederalkyl, Hydroxyniederalkyl, Niederalkylaminoniederalkyl, Aminoniederalkyl, Cycloalkyl, Cycloalkylniederalkyl, Niederalkoxyniederalkyl, Aryl, Arylniederalkyl oder Halogenniederalkyl darstellen;
R₈ H, Niederalkyl, Hydroxyniederalkyl, Niederalkylamino; Niederalkylaminoniederalkyl, Aminoniederalkyl, Cycloalkyl, Cycloalkylniederalkyl, Niederalkenyl, Niederalkoxy, Niederalkoxyniederalkyl, Halogenniederalkyl, Aryl oder Arylniederalkyl darstellt;
R₉ H, Niederalkyl oder Halogenniederalkyl darstellt;
R₁₀ H, Niederalkyl, Halogenniederalkyl oder Niederalkoxy darstellt;
R₁₈, R₁₉ und R₂₀ H darstellen.

3. Verfahren nach einem der vorhergehenden Ansprüche, **dadurch gekennzeichnet, dass** R₁ eine Ethylgruppe darstellt.

4. Verfahren nach einem der vorhergehenden Ansprüche, **dadurch gekennzeichnet, dass** R₂ und R₃ unabhängig voneinander H, Niederalkyl, Halogen, Halogenniederalkyl oder -(CH₂)ₘOR₆ darstellen oder R₂ und R₃ zusammen Methylendioxy oder Ethylendioxy bilden; und R₄ und R₅ unabhängig voneinander H, Niederalkyl, -(CH₂)ₘNR₆R₇ oder nicht substituiertes oder durch ein Niederalkyl substituiertes -(CH₂)ₙ[N=X] darstellen.

5. Verfahren nach einem der vorhergehenden Ansprüche, **dadurch gekennzeichnet, dass** R₄ H oder -(CH₂)ₘNR₆R₇ darstellt, worin R₆ und R₇ unabhängig voneinander H oder ein Niederalkyl darstellen und R₅ H, ein Niederalkyl oder substituiertes oder nicht substituiertes -(CH₂)ₙ[N=X] darstellt und [N=X] eine Piperazinyl- oder Morpholinylgruppe darstellt und der Substituent ein Niederalkyl ist.

6. Verfahren nach einem der vorhergehenden Ansprüche, **dadurch gekennzeichnet, dass** R₂ H oder Halogen darstellt und R₃ H, Niederalkyl, Halogen oder OR₆ darstellt, worin R₆ H, Niederalkyl oder Arylniederalkyl darstellt.

7. Verfahren nach einem der vorhergehenden Ansprüche, **dadurch gekennzeichnet, dass** R₂ H, Chlor oder Fluor darstellt und R₃ H, Fluor, Chlor, Methyl oder Methoxy darstellt.

8. Verbindungen der Formel O 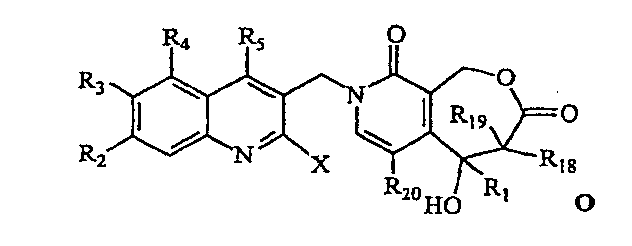 in der R₁, R₂, R₃, R₄, R₅, R₁₈, R₁₉, R₂₀ und X die in Anspruch 1 angegebene Bedeutung haben.

9. Verwendung der Verbindungen der Formel O, wie sie in Anspruch 8 definiert sind, für die Herstellung der Verbindungen der Formel I 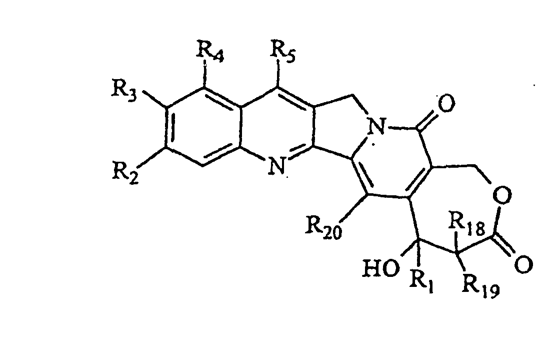 in der R₁, R₂, R₃, R₄, R₅, R₁₈, R₁₉ und R₂₀ die in Anspruch 1 angegebene Bedeutung haben, oder ein pharmazeutisch annehmbares Salz davon,
**dadurch gekennzeichnet, dass** man die Verbindung der allgemeinen Formel O, wie sie in Anspruch 8 definiert ist, cyclisiert, um die Verbindung der allgemeinen Formel I, wie sie oben definiert ist, zu erhalten.

10. Verwendung nach Anspruch 9 für die Herstellung einer Verbindung der Formel (I), **dadurch gekennzeichnet, dass** diese Verbindung der folgenden Formel entspricht: 5-Ethyl-9,10-difluor-4,5-dihydro-5-hydroxy-1*H*-oxepino[3',4':6,7] indolizino [1,2-*b*] chinolin-3,15(4*H*,13*H*)-dion.

## Claims

1. Preparation process of compounds of formula O 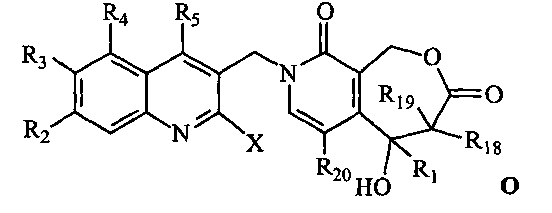 in racemic or enantiomeric form or any combinations of these forms, in which
R₁ represents a lower alkyl, a lower alkenyl, a lower alkynyl, a lower haloalkyl, a lower alkoxy lower alkyl or lower alkylthio lower alkyl ;
R₂, R₃ and R₄ represent, independently, H, halo, lower halo alkyl, lower alkyl, lower alkenyl, cyano, lower cyano alkyl, nitro, lower nitro alkyl, amido, lower amido alkyl, hydrazino, lower hydrazino alkyl, azido, lower azido alkyl, -(CH₂)ₘNR₆R₇, -(CH₂)ₘOR₆, -(CH₂)ₘSR₆, -(CH₂)ₘCO₂R₆, -(CH2)ₘNR₆C(O)R₈, -(CH₂)ₘC(O)R₈, -(CH₂)ₘOC(O)R₈, -O(CH₂)ₘNR₆R₇, -OC(O)NR₆R₇, -OC(O)(CH₂)ₘCO₂R₆, or substituted or non substituted -(CH₂)ₙ[N=X], -OC(O)[N=X], -(CH₂)ₘOC(O)[N=X], aryl or lower aryl-alkyl, in which the substituent is a lower alkyl, halo, nitro, amino, lower alkylamino, lower haloalkyl, lower hydroxy alkyl, lower alkoxy or lower alkoxy lower alkyl, or R₂ and R₃ form together a chain with 3 or 4 members in which the elements of the chain are selected from the group constituted by CH, CH₂, O, S, N or NR₉;
R₅ represents H, halo, lower halo alkyl, lower alkyl, lower alkoxy, lower alkoxy lower alkyl, lower alkylthio lower alkyl, cycloalkyl, lower cycloalkyl alkyl, cyano, cyano alkyl, lower alkyl sulphonyl lower alkyl, lower hydroxy alkyl, nitro, -(CH₂)ₘC(O)R₈, -(CH₂)ₘNR₆C(O)R₈, -(CH₂)ₘNR₆R₇, -(CH₂)ₘN(CH₃)(CH₂)ₙNR₆R₇, -(CH₂)ₘOC(O)R₈, -(CH₂)ₘOC(O)NR₆R₇, -(CH₂)ₘS(O)_{q}R₁₁, -(CH₂)ₘP(O)R₁₂R₁₃, -(CH₂)₂P(S)R₁₂R₁₃, or a substituted or non substituted -(CH₂)ₙ[N=X], -OC(O)[N=X], -(CH₂)ₘOC(O)[N=X], aryl or lower aryl alkyl radical, in which the substituent is a lower alkyl, halo, nitro, amino, lower alkyl amino, lower halo alkyl, lower hydroxy alkyl, lower alkoxy or lower alkoxy lower alkyl ;
R₆ and R₇ represent, independently, H, a lower alkyl, lower hydroxy alkyl, lower alkyl amino lower alkyl, lower amino alkyl, cycloalkyl, lower cycloalkyl alkyl, lower alkenyl, lower alkoxy lower alkyl, lower halo alkyl, or substituted or non substituted aryl or lower aryl alkyl radical, in which the substituent is a lower alkyl, halo, nitro, amino, lower alkyl amino, lower halo alkyl, lower hydroxy alkyl, lower alkoxy or lower alkoxy lower alkyl ;
R₈ represents H, a lower alkyl, lower hydroxy alkyl, amino, lower alkyl amino, lower alkyl amino lower alkyl, lower amino alkyl, cycloalkyl, lower cycloalkyl alkyl, lower alkenyl, lower alkoxy, lower alkoxy lower alkyl, lower halo alkyl, or the substituted or non substituted aryl or lower aryl alkyl radical, in which the substituent is a lower alkyl, halo, nitro, amino, lower alkyl amino, lower halo alkyl, lower hydroxy alkyl, lower alkoxy or lower alkoxy lower alkyl ;
R₉ represents H, a lower alkyl, lower halo alkyl, aryl, or aryl substituted by one or more groups chosen from lower alkyl, halo, nitro, amino, lower alkyl amino, lower halo alkyl, lower hydroxy alkyl, lower alkoxy or lower alkoxy lower alkyl ;
R₁₀ represents H, a lower alkyl, lower halo alkyl, lower alkoxy, aryl or aryl substituted by one or more groups chosen from lower alkyl, lower halo alkyl, lower hydroxy alkyl or lower alkoxy lower alkyl ;
R₁₁ represents a lower alkyl, aryl, -(CH₂)ₘOR₁₄, -(CH₂)ₘSR₁₄, -CH₂)₂NR₁₄R₁₅ or (CH₂)ₘ[N=X] ;
R₁₂ and R₁₃ represent, independently, a lower alkyl, aryl, lower alkoxy, aryloxy or amino;
R₁₄ and R₁₅ represent, independently, H, lower alkyl or aryl;
R₁₈ and R₁₉ represent, independently, H, halo, lower alkyl, lower alkoxy or hydroxy;
R₂₀ represents H or halo;
m is an integer comprised between 0 and 6;
n is 1 or 2; and
q represents an integer from 0 to 2;
[N=X] represents a heterocyclic group with 4 to 7 members, X representing the chain necessary to complete said heterocyclic group and selected from the group constituted by O, S, CH₂, CH, N, NR₉ and COR₁₀ ;
X represents a halogen atom ; or a pharmaceutically acceptable salt thereof,
with the proviso that the term "lower" referring to alkyl, alkylthio and alkoxy radicals, means a radical containing from 1 to 6 carbon atoms, and referring to alkenyl or alkynyl radicals, means a radical containing from 2 to 6 carbon atoms,
**characterized in that** a compound of general formula M 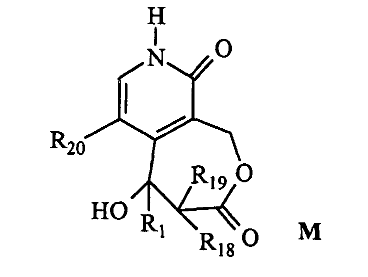 in which R₁, R₁₈, R₁₉ and R₂₀ have the meaning indicated above, is coupled with 2-halo-3-quinoline-methanol of general formula N 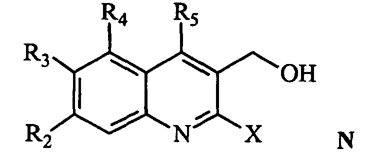 in which R₂, R₃, R₄, R₅ and X have the meaning indicated above, in order to produce the compound of formula O.

2. Preparation process according to claim 1, **characterized in that**
R₁ represents a lower alkyl, a lower alkenyl, a lower halo alkyl or a lower alkoxy lower alkyl ;
R₂, R₃ and R₄ represent, independently, H, a halo, lower halo alkyl, lower alkyl, nitro, amido, lower amido alkyl, hydrazino, lower hydrazino alkyl, azido, lower azido alkyl, -(CH2)ₘNR₆,R₇, -(CH₂)ₘOR₆, -(CH₂)ₘSR₆, -(CH₂)ₘC(O)R₈, -OC(O)NR₆R₇, substituted or non substituted -(CH₂)ₙ[N=X] or -(CH₂)ₘOC(O)[N=X], or -OC(O)[N=X], or R₂ and R₃ form together a chain with 3 or 4 members in which the said elements of the chain are selected from the group constituted by CH, CH₂, O, S, N or NR₉;
R₅ represents H, halo, lower halo alkyl, lower alkyl, lower alkoxy, lower alkoxy lower alkyl, lower alkylthio lower alkyl, lower hydroxy alkyl, nitro, -(CH₂)ₘC(O)R₈, -(CH₂)ₘNR₆C(O)R₈, -(CH₂)ₘNR₆R₇, -(CH₂)ₘN(CH₃)(CH₂)ₙNR₆R₇, -(CH₂)ₘOC(O)R₈, -(CH₂)ₘOC(O)NR₆R₇, substituted or non substituted -(CH₂)ₙ[N=X] or -OC(O)[N=X], or -(CH₂)ₘOC(O)[N=X] ;
R₆ and R₇ represent, independently, H, a lower alkyl, lower hydroxy alkyl, lower alkyl amino lower alkyl, lower amino alkyl, cycloalkyl, lower cycloalkyl alkyl, lower alkoxy lower alkyl, aryl, lower aryl alkyl or lower halo alkyl;
R₈ represents H, a lower alkyl, lower hydroxy alkyl, lower alkyl amino, lower alkyl amino lower alkyl, lower amino alkyl, cycloalkyl, lower cycloalkyl alkyl, lower alkenyl, lower alkoxy, lower alkoxy lower alkyl, lower halo alkyl, aryl or lower aryl alkyl;
R₉ represents H, a lower alkyl or lower halo alkyl;
R₁₀ represents H, a lower alkyl, lower halo alkyl or lower alkoxy;
R₁₈, R₁₉ and R₂₀ represent H.

3. Preparation process according to any of the preceding claims, **characterized in that** R₁ represents the ethyl group.

4. Preparation process according to any of the preceding claims, **characterized in that** R₂ and R₃ represent, independently, H, a lower alkyl, halo, lower halo alkyl or -(CH₂)ₘOR₆, or R₂ and R₃, together, form a methylenedioxy or an ethylenedioxy; and R₄ and R₅ represent, independently, H, a lower alkyl, -(CH₂)ₘNR₆R₇ or -(CH₂)ₙ[N=X] non substituted or substituted by a lower alkyl.

5. Preparation process according to any of the preceding claims, **characterized in that** R₄ represents H or -(CH₂)ₘNR₆R₇ in which R₆ and R₇ represent, independently, H or a lower alkyl, and R₅ represents H, a lower alkyl or substituted or non substituted -(CH₂)ₙ[N=X], and [N=X] represents the piperazinyl or morpholinyl group and said substituent is a lower alkyl.

6. Preparation process according to any of the preceding claims, **characterized in that** R₂ represents H or a halo; and R₃ represents H, a lower alkyl, halo, or OR₆ in which R₆ represents H, a lower alkyl or lower aryl alkyl.

7. Preparation process according to any of the preceding claims, **characterized in that** R₂ represents H, chloro or fluoro; and R₃ represents H, fluoro, chloro, methyl or methoxy.

8. Compounds of formula O 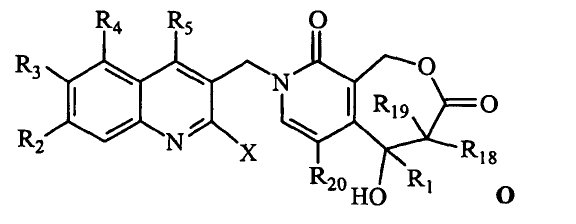 in which R₁, R₂, R₃, R₄, R₅, R₁₈, R₁₉, R₂₀ and X have the meaning indicated in claim 1.

9. Use of compounds of formula O as defined in claim 8, for the preparation process of compounds of formula I 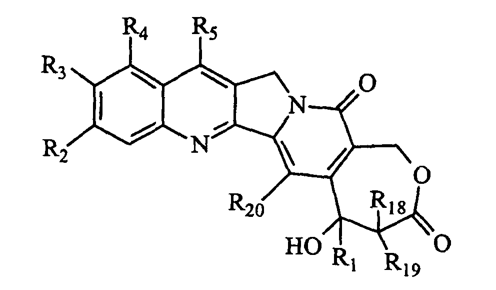 in which R₁, R₂, R₃, R₄, R₅, R₁₈, R₁₉ and R₂₀ have the meaning indicated in claim 1, or a pharmaceutically acceptable salt thereof,
**characterized in that** the compound of general formula O as defined in claim 8, is cyclized in order to obtain the compound of general formula I as defined above.

10. Use according to claim 9, for the preparation process of a compound of formula (I), **characterized in that** said compound corresponds to the following formula : 5-ethyl-9,10-difluoro-4,5-dihydro-5-hydroxy-1*H*-oxepino[3',4':6,7]indolizino[1,2-*b*] quinoline-3,15(4*H*,13*H*)-dione.
